# EUROPEAN PATENT SPECIFICATION

(11) **EP 4 048 215 B1**
(45) Date of publication and mention of the grant of the patent: **01.04.2026**
(21) Application number: 20878467.8
(22) Date of filing: 22.10.2020
(51) Int. Cl.: A61M 1/00, A61F 13/00, A61F 13/05

(54) **NEGATIVE PRESSURE WOUND THERAPY (NPWT) BANDAGE**
UNTERDRUCKWUNDTHERAPIE (NPWT)-VERBAND
BANDAGE POUR THÉRAPIE DE PLAIE PAR PRESSION NÉGATIVE (NPWT)

(30) Priority: 22.10.2019 US 201962924386 P; 22.10.2019 US 201962924290 P; 22.10.2019 US 201962924432 P; 20.03.2020 US 202062992667 P; 22.09.2020 US 202063081690 P
(43) Date of publication of application: 31.08.2022
(73) Proprietor: Stryker Corporation, Portage, MI 49002 (US)
(72) Inventor: CORNHILL, John Frederick, Fort Lauderdale, FL 33305 (US); VAN DER LEEST, Machiel, 75001 Paris (FR); CORWIN, Russell, Elmsford, NY 10523 (US)
(74) Representative: Cullinane, Marietta Bettina
(86) International application number: PCT/US2020/056871
(87) International publication number: WO 2021/081210

(56) References cited:
- WO-A1-2019/113275
- WO-A1-2019/113275
- US-A- 5 549 584
- US-A1- 2013 304 007
- US-A1- 2013 304 007
- US-A1- 2015 202 354
- US-A1- 2016 199 230
- US-A1- 2017 143 552
- US-A1- 2017 304 512
- US-A1- 2018 168 869
- US-A1- 2018 272 052
- US-A1- 2018 272 052
- US-A1- 2019 269 835
- US-A2- 2011 087 177
- US-A2- 2011 087 177
- US-B2- 7 779 625

## Description

### Field Of The Invention

This invention relates to negative pressure wound therapy (NPWT) bandages.

### Background Of The Invention

Bandages are used to provide wound care during healing. More particularly, bandages generally provide a covering for a wound so as to shield the wound from contaminants and microbes during healing. Most bandages also provide a closure feature to help hold the edges of the wound in close apposition during healing. Bandages also frequently include gauze or the like to receive exudates emerging from the wound during healing.

Negative pressure wound therapy (NPWT) bandages apply a negative pressure to a wound during healing. This negative pressure helps reduce the likelihood of contaminants and microbes entering the wound during healing, helps draw exudates from the wound during healing, and can promote beneficial biological responses at the wound site. More particularly, NPWT bandages typically comprise (i) an absorbent dressing configured to make a fully-sealed chamber around the perimeter of a wound ("the wound chamber"), (ii) a source of negative pressure, and (iii) a conduit extending between the fully-sealed wound chamber and the source of negative pressure. As a result of this construction, the absorbent dressing can be applied to a wound so as to create a fully-sealed chamber around the perimeter of the wound, and the source of negative pressure can apply a negative pressure to the fully-sealed wound chamber, such that any contaminants and microbes present at the wound site are drawn away from the wound, exudates are drawn out of the wound, and beneficial biological responses are promoted at the wound site.

US 2018/272052 A1 discloses systems, assemblies, and methods for providing negative-pressure therapy to a tissue site. The system can include an absorbent and a sealing layer configured to cover the absorbent. The system can also include a blister fluidly coupled to the absorbent. The blister may have a collapsed position and an expanded position. A first check valve may be fluidly coupled to the absorbent and the blister and configured to prevent fluid flow from the blister into the absorbent if the blister is moved from the expanded position to the collapsed position. A second check valve may be fluidly coupled to the blister and the ambient environment and configured to prevent fluid flow from the ambient environment into the blister if the blister is moved from the collapsed position to the expanded position.

Most NPWT bandages are part of a large, complex NPWT system, in the sense that (i) the absorbent dressings are generally fairly large (e.g., they are sized to cover large open wounds), (ii) the sources of negative pressure are generally fairly large, and formed and located separate from the absorbent dressings (e.g., the sources of negative pressure typically comprise electrically-powered suction pumps or vacuum canisters), and (iii) the NPWT systems generally require substantial training to use. These NPWT systems also tend to be quite expensive.

Efforts have been made to provide a small, simplified and less expensive NPWT bandage where the source of negative pressure is integrated with the absorbent dressing. By way of example but not limitation, efforts have been made to provide an NPWT bandage where a manually-operated suction pump is integrated into the absorbent dressing. Unfortunately, current NPWT bandages integrating a suction pump with the absorbent dressing tend to suffer from a variety of deficiencies, e.g., they have a complex design, and/or are expensive, and/or are complicated to use, and/or are bulky (including having a high profile), and/or cause additional trauma to the wound during use, and/or have poor pump efficiency, and/or lack a way of indicating the level of negative pressure created, and/or lack a way of limiting the level of negative pressure created, etc. In this latter respect it should be appreciated that where too high a level of negative pressure is created, the NPWT bandage can cause trauma to the patient, e.g., blistering, capillary leakage, etc.

Thus there is a need for a new and improved NPWT bandage which is simple, inexpensive, easy-to-use, small in size (including having a low profile), is atraumatic to the wound during use, has improved pump efficiency, incorporates an automatic pressure indicator for indicating the level of negative pressure created, and provides an automatic pressure limiter for limiting the level of negative pressure created.

### Summary Of The Invention

These and other objects of the invention are addressed by the provision and use of a new and improved NPWT bandage which is simple, inexpensive, easy-to-use, small in size (including having a low profile), is atraumatic to the wound during use, has improved pump efficiency, incorporates an automatic pressure indicator for indicating the level of negative pressure created, and provides an automatic pressure limiter for limiting the level of negative pressure created.

The NPWT according to the invention comprises the features of claim 1. Preferred embodiments are described in dependent claims 2 to 9.

In examples of the disclosure, not forming part of the invention as claimed, the negative pressure wound therapy (NPWT) bandage for applying negative pressure to a wound, comprises:
a membrane configured for disposition over a wound so as to form a wound chamber between said membrane and the wound, said membrane comprising a wound-side surface, an atmosphere-side surface, and an opening extending through said membrane from said wound-side surface to said atmosphere-side surface; and
a pump assembly carried by said membrane, said pump assembly comprising:
   a pump body comprising a wall structure disposed about a pump chamber, wherein at least a portion of said wall structure is resilient;
   a wound-side passageway extending through said wall structure and communicating with the wound chamber through said opening formed in said membrane;
   a wound-side one-way valve disposed in said wound-side passageway, said wound-side one-way valve being configured to allow fluid to flow through said wound-side passageway from the wound chamber to said pump chamber but to prevent fluid from flowing through said wound-side passageway from said pump chamber to the wound chamber;
   an atmosphere-side passageway extending through said wall structure and connecting said pump chamber and the atmosphere; and
   an atmosphere-side one-way valve disposed in said atmosphere-side passageway, said atmosphere-side one-way valve being configured to allow fluid to flow through said atmosphere-side passageway from said pump chamber to the atmosphere but to prevent fluid from flowing through said atmosphere-side passageway from the atmosphere to said pump chamber;
such that when a compressive force is applied to said wall structure of said pump body, fluid within said pump chamber will be forced out of said pump chamber via said atmosphere-side passageway, and when the compressive force applied to said wall structure of said pump body is thereafter reduced, fluid within the wound chamber will be drawn into said pump chamber through said wound-side passageway.

Preferably, the NPWT bandage is configured so that when the pressure differential between the pressure of the fluid within said pump chamber and atmospheric pressure is below a predetermined threshold, said pump body of said pump assembly will assume a substantially fully expanded configuration, and when said pressure differential between the pressure of the fluid within said pump chamber and atmospheric pressure is above said predetermined threshold, said pump body of said pump assembly will assume a substantially fully collapsed configuration.

Even more preferably, the NPWT bandage is configured so that said pump body abruptly changes state between said substantially fully expanded configuration and said substantially fully collapsed configuration, and between said substantially fully collapsed configuration and said substantially fully expanded configuration, as said pressure differential crosses said predetermined threshold so as to effectively constitute a substantially "binary state" device.

For illustration purposes only, a method for applying negative pressure to a wound is described, the method comprising:
providing a negative pressure wound therapy (NPWT) bandage comprising:
   a membrane configured for disposition over a wound so as to form a wound chamber between said membrane and the wound, said membrane comprising a wound-side surface, an atmosphere-side surface, and an opening extending through said membrane from said wound-side surface to said atmosphere-side surface; and
   a pump assembly carried by said membrane, said pump assembly comprising:
      a pump body comprising a wall structure disposed about a pump chamber, wherein at least a portion of said wall structure is resilient;
      a wound-side passageway extending through said wall structure and communicating with the wound chamber through said opening formed in said membrane;
      a wound-side one-way valve disposed in said wound-side passageway, said wound-side one-way valve being configured to allow fluid to flow through said wound-side passageway from the wound chamber to said pump chamber but to prevent fluid from flowing through said wound-side passageway from said pump chamber to the wound chamber;
      an atmosphere-side passageway extending through said wall structure and connecting said pump chamber and the atmosphere; and
      an atmosphere-side one-way valve disposed in said atmosphere-side passageway, said atmosphere-side one-way valve being configured to allow fluid to flow through said atmosphere-side passageway from said pump chamber to the atmosphere but to prevent fluid from flowing through said atmosphere-side passageway from the atmosphere to said pump chamber;
   such that when a compressive force is applied to said wall structure of said pump body, fluid within said pump chamber will be forced out of said pump chamber via said atmosphere-side passageway, and when the compressive force applied to said wall structure of said pump body is thereafter reduced, fluid within the wound chamber will be drawn into said pump chamber through said wound-side passageway;
positioning said negative pressure wound therapy (NPWT) bandage over the wound so as to form a wound chamber between said membrane and the wound; and
applying a compressive force to said wall structure of said pump body, and thereafter reducing the compressive force applied to said wall structure of said pump body, so as to apply negative pressure to the wound.

In examples of the disclosure, not forming part of the invention as claimed, the negative pressure wound therapy (NPWT) bandage for applying negative pressure to a wound comprises:
a membrane configured for disposition over a wound so as to form a wound chamber between said membrane and the wound, said membrane comprising a wound-side surface, an atmosphere-side surface, and an opening extending through said membrane from said wound-side surface to said atmosphere-side surface; and
a pump carried by said membrane and comprising a wall chamber disposed about a pump chamber, wherein at least a portion of said wall chamber is resilient, and further wherein said pump chamber communicates with the wound chamber through said opening formed in said membrane;
wherein no part of said pump chamber is defined by the wound.

In another preferred form the negative pressure wound therapy (NPWT) bandage for applying negative pressure to a wound comprises:
a membrane configured for disposition over a wound so as to form a wound chamber between said membrane and the wound, said membrane comprising a wound-side surface, an atmosphere-side surface, and an opening extending through said membrane from said wound-side surface to said atmosphere-side surface; and
a pump carried by said membrane and comprising a wall chamber disposed about a pump chamber, wherein at least a portion of said wall chamber is resilient, and further wherein said pump chamber communicates with the wound chamber through said opening formed in said membrane;
wherein said pump does not apply positive pressure to the wound. In examples of the disclosure, not forming part of the invention as claimed, the negative pressure wound therapy (NPWT) bandage for applying negative pressure to a wound, comprises:
   a membrane configured for disposition over a wound so as to form a wound chamber between said membrane and the wound, said membrane comprising a wound-side surface, an atmosphere-side surface, and an opening extending through said membrane from said wound-side surface to said atmosphere-side surface; and
   a pump carried by said membrane and comprising a wall chamber disposed about a pump chamber, wherein at least a portion of said wall chamber is resilient, and further wherein said pump chamber communicates with the wound chamber through said opening formed in said membrane;
   wherein said pump is connected to the wound chamber such that a reduction in the volume of the pump chamber does not cause a change in pressure in the wound chamber.

In examples of the disclosure, not forming part of the invention as claimed, the negative pressure wound therapy (NPWT) bandage is constructed so that it also provides a negative pressure decay "warning state" indicator.

In examples of the disclosure, not forming part of the invention as claimed, the negative pressure wound therapy (NPWT) bandage for applying negative pressure to a wound, the NPWT bandage comprises:
a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface; and
a pump assembly carried by the membrane, the pump assembly comprising:
   a pump body comprising a wall structure disposed about a pump chamber, wherein at least a portion of the wall structure is resilient, and further wherein the pump chamber communicates with the wound chamber through the opening formed in the membrane; and
   an atmosphere-side passageway extending through the wall structure so as to fluidically connect the pump chamber to the atmosphere;
   such that when a compressive force is applied to the wall structure of the pump body, fluid within the pump chamber is forced out of the pump chamber via the atmosphere-side passageway, and when the compressive force applied to the wall structure of the pump body is thereafter reduced, fluid within the wound chamber is drawn into the pump chamber through the opening formed in the membrane;
wherein when the pressure differential between the pressure of the fluid within the pump chamber and the atmospheric pressure is below a predetermined threshold, the pump body will assume a substantially fully expanded configuration, whereby to indicate that negative pressure within the wound chamber is below the predetermined threshold, and when the pressure differential between the pressure of the fluid within the pump chamber and the atmospheric pressure is above the predetermined threshold, the pump body will assume a substantially fully collapsed configuration, whereby to indicate that the negative pressure within the wound chamber is above the predetermined threshold; and
further wherein the pump body is configured to provide a visual indicator that the negative pressure within the wound chamber is falling, but has not fallen enough to go below the predetermined threshold and cause a transformation of the pump body into its substantially fully expanded configuration.

For illustration purposes only, a method for applying negative pressure to a wound is described, the method comprising:
providing a negative pressure wound therapy (NPWT) bandage comprising:
   a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface; and
   a pump assembly carried by the membrane, the pump assembly comprising:
      a pump body comprising a wall structure disposed about a pump chamber, wherein at least a portion of the wall structure is resilient, and further wherein the pump chamber communicates with the wound chamber through the opening formed in the membrane; and
      an atmosphere-side passageway extending through the wall structure so as to fluidically connect the pump chamber to the atmosphere;
      wherein when a compressive force is applied to the wall structure of the pump body, fluid within the pump chamber is forced out of the pump chamber via the atmosphere-side passageway, and when the compressive force applied to the wall structure of the pump body is thereafter reduced, fluid within the wound chamber is drawn into the pump chamber through the opening formed in the membrane;
      wherein when the pressure differential between the pressure of the fluid within the pump chamber and the atmospheric pressure is below a predetermined threshold, the pump body assumes a substantially fully expanded configuration, whereby to indicate that negative pressure within the wound chamber is below the predetermined threshold, and when the pressure differential between the pressure of the fluid within the pump chamber and the atmospheric pressure is above the predetermined threshold, the pump body assumes a substantially fully collapsed configuration, whereby to indicate that the negative pressure within the wound chamber is above the predetermined threshold; and
      further wherein the pump body is configured to provide a visual indicator that the negative pressure within the wound chamber is falling, but has not fallen enough to go below the predetermined threshold and cause a transformation of the pump body into its substantially fully expanded configuration;
positioning the NPWT bandage over the wound so as to form the wound chamber between the membrane and the wound; and
applying a compressive force to the wall structure of the pump body, and thereafter reducing the compressive force applied to the wall structure of the pump body, so as to apply negative pressure to the wound.

According to the invention, the negative pressure wound therapy (NPWT) bandage includes a canister for collecting exudates.

According to the invention, there is provided a negative pressure wound therapy (NPWT) bandage for applying negative pressure to a wound, the NPWT bandage comprising:
a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface;
a pump assembly carried by the membrane, the pump assembly comprising:
   a pump body comprising a wall structure disposed about a pump chamber, wherein at least a portion of the wall structure is resilient;
   a wound-side passageway extending through the wall structure and communicating with the wound chamber; and
   an atmosphere-side passageway extending through the wall structure and connecting the pump chamber to the atmosphere; and
a canister configured to be in fluid communication with the wound chamber and the pump assembly, the canister comprising an interior chamber for collecting exudates exiting the wound chamber; wherein the pump assembly is configured to generate a negative air pressure within the wound chamber such that exudates from the wound chamber will flow out of the wound chamber and into the interior chamber of the canister.

For illustration purposes only, a method for applying negative pressure to a wound is described, the method comprising:
providing a negative pressure wound therapy (NPWT) bandage comprising:
   a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface;
   a pump assembly carried by the membrane, the pump assembly comprising:
      a pump body comprising a wall structure disposed about a pump chamber, wherein at least a portion of the wall structure is resilient;
      a wound-side passageway extending through the wall structure and communicating with the wound chamber; and
      an atmosphere-side passageway extending through the wall structure and connecting the pump chamber to the atmosphere; and
   a canister configured to be in fluid communication with the wound chamber and the pump assembly, the canister comprising an interior chamber for collecting exudates exiting the wound chamber;
   wherein the pump assembly is configured to generate a negative air pressure within the wound chamber such that exudates from the wound chamber will flow out of the wound chamber and into the interior chamber of the canister;
positioning the NPWT bandage over the wound so as to form a wound chamber between the membrane and the wound; and
generating a negative air pressure within the wound chamber so that exudates flow from the wound chamber and into the interior chamber of the canister.

In another preferred form, the negative pressure wound therapy (NPWT) bandage includes a luer lock for suction attachment.

Disclosed is also a negative pressure wound therapy (NPWT) bandage for applying negative pressure to a wound, the NPWT bandage comprising:
a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface;
a pump assembly carried by the membrane, the pump assembly comprising:
   a pump body comprising a wall structure disposed about a pump chamber, wherein at least a portion of the wall structure is resilient;
   a wound-side passageway extending through the wall structure and communicating with the wound chamber through the opening in the membrane; and
   an atmosphere-side passageway extending through the wall structure and connecting the pump chamber to the atmosphere; and
a suction source configured to be connected to the pump assembly for establishing negative air pressure within the wound chamber;
wherein when the negative air pressure within the wound chamber is below a predetermined threshold, the pump body will assume a substantially fully expanded configuration, and when the negative air pressure within the wound chamber is above the predetermined threshold, the pump body will assume a substantially fully collapsed configuration.

For illustration purposes only, a method for applying negative pressure to a wound is described, the method comprising:
providing a negative pressure wound therapy (NPWT) bandage comprising:
   a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface;
   a pump assembly carried by the membrane, the pump assembly comprising:
      a pump body comprising a wall structure disposed about a pump chamber, wherein at least a portion of the wall structure is resilient;
      a wound-side passageway extending through the wall structure and communicating with the wound chamber through the opening in the membrane;
      an atmosphere-side passageway extending through the wall structure and connecting the pump chamber and the atmosphere; and
   a suction source configured to be connected to the pump assembly for establishing negative air pressure within the wound chamber;
   wherein when the negative air pressure within the wound chamber is below a predetermined threshold, the pump body will assume a substantially fully expanded configuration, and when the negative air pressure within the wound chamber is above the predetermined threshold, the pump body will assume a substantially fully collapsed configuration;
positioning the NPWT bandage over the wound so as to form a wound chamber between the membrane and the wound; and
connecting the suction source to the pump assembly to generate a negative air pressure within the wound chamber.

In examples of the disclosure, not forming part of the invention as claimed, the negative pressure wound therapy (NPWT) bandage for applying negative pressure to a wound comprises:
a membrane configured for disposition over a wound so as to form a wound chamber between the membrane and the wound, the membrane comprising a wound-side surface, an atmosphere-side surface, and an opening extending through the membrane from the wound-side surface to the atmosphere-side surface;
a pump assembly carried by the membrane, the pump assembly comprising:
   a pump body comprising a wall structure disposed about a pump chamber, wherein at least a portion of the wall structure is resilient;
   a wound-side passageway extending through the wall structure and communicating with the wound chamber through the opening in the membrane; and
   an atmosphere-side passageway extending through the wall structure and connecting the pump chamber to the atmosphere; and
a connector for connecting a suction source to the pump assembly for establishing negative air pressure within the wound chamber;
wherein when the negative air pressure within the wound chamber is below a predetermined threshold, the pump body will assume a substantially fully expanded configuration, and when the negative air pressure within the wound chamber is above the predetermined threshold, the pump body will assume a substantially fully collapsed configuration.

In another preferred form, the negative pressure wound therapy (NPWT) bandage is provided with a pump assembly that is laterally offset from a wound while remaining in fluid communication with the wound.

In examples of the disclosure, not forming part of the invention as claimed, the negative pressure wound therapy (NPWT) bandage for applying negative pressure to a wound, the NPWT bandage comprises:
a membrane comprising a first portion configured for disposition over a wound so as to form a wound chamber between the membrane and the wound, and a second portion configured to extend laterally away from the wound, the membrane comprising a wound-side surface, an atmosphere-side surface, and an opening extending from the wound-side surface to the atmosphere-side surface, the opening being in fluid communication with the wound chamber; and
a pump assembly carried by the second portion of the membrane, the pump assembly comprising:
   a pump body comprising a wall structure disposed about a pump chamber, wherein at least a portion of the wall structure is resilient;
   a wound-side passageway extending through the wall structure and communicating with the wound chamber through the opening in the membrane; and
   an atmosphere-side passageway extending through the wall structure and connecting the pump chamber to the atmosphere;
wherein the pump assembly is configured to generate a negative air pressure within the wound chamber.

For illustration purposes only, a method for applying negative pressure to a wound is described, the method comprising:
providing a negative pressure wound therapy (NPWT) bandage comprising:
   a membrane comprising a first portion configured for disposition over a wound so as to form a wound chamber between the membrane and the wound, and a second portion configured to extend laterally away from the wound, the membrane comprising a wound-side surface, an atmosphere-side surface, and an opening extending from the wound-side surface to the atmosphere-side surface, the opening being in fluid communication with the wound chamber; and
   a pump assembly carried by the second portion of the membrane, the pump assembly comprising:
      a pump body comprising a wall structure disposed about a pump chamber, wherein at least a portion of the wall structure is resilient;
      a wound-side passageway extending through the wall structure and communicating with the wound chamber through the opening formed in the membrane; and
      an atmosphere-side passageway extending through the wall structure and connecting the pump chamber to the atmosphere;
   wherein the pump assembly is configured to generate a negative air pressure within the wound chamber;
positioning the first portion of the NPWT bandage over the wound so as to form a wound chamber between the membrane and the wound and positioning the second portion of the membrane on anatomy that is laterally offset from the wound; and
generating a negative air pressure within the wound chamber.

In another preferred form the negative pressure wound therapy bandage for covering a wound, comprises a transparent top layer which captures a layer of foam against the wound, wherein the foam layer is provided with a series of windows which (i) allow direct visualization of the wound, and (ii) provide a metric along the length of the bandage to indicate whether the bandage needs to be replaced. The foam layer also comprises perimeter serrations which allow the bandage to conform to a curved wound.

In examples of the disclosure, not forming part of the invention as claimed, the negative pressure wound therapy (NPWT) bandage for applying negative pressure to a wound comprises:
a membrane configured for disposition over a wound so as to form a wound chamber between the membrane and the wound, the membrane comprising a wound-side surface, an atmosphere-side surface, and an opening extending from the wound-side surface to the atmosphere-side surface, the opening being in fluid communication with the wound chamber;
   wherein the membrane comprises a transparent layer and an absorbent layer, wherein the absorbent layer is disposed on the wound-side of the membrane;
   wherein the absorbent layer comprises a plurality of windows extending through the absorbent layer; and
   further wherein the transparent layer of the membrane and the plurality of windows of the absorbent layer of the membrane permit visualization of the wound through the membrane; and
a pump assembly carried by the membrane, the pump assembly comprising:
   a pump body comprising a wall structure defining a pump chamber, wherein at least a portion of the wall structure is resilient;
   a wound-side passageway extending through the wall structure and communicating with the wound chamber through the opening in the membrane; and
   an atmosphere-side passageway extending through the wall structure and connecting the pump chamber to the atmosphere;
   wherein the pump assembly is configured to generate a negative air pressure within the wound chamber.

In examples of the disclosure, not forming part of the invention as claimed, the bandage for covering a wound releasing exudate, the bandage comprises:
a membrane configured for disposition over a wound so as to form a wound chamber between the membrane and the wound, the membrane comprising a wound-side surface, an atmosphere-side surface, and an opening extending from the wound-side surface to the atmosphere-side surface, the opening being in fluid communication with the wound chamber;
wherein the membrane comprises a transparent layer and an absorbent layer, wherein the absorbent layer is disposed on the wound-side of the membrane;
wherein the absorbent layer comprises a plurality of windows extending through the absorbent layer; and
further wherein the transparent layer of the membrane and the plurality of windows of the absorbent layer of the membrane permit visualization of the wound through the membrane.

For illustration purposes only, a method for applying negative pressure to a wound, the method comprising:
providing a negative pressure wound therapy (NPWT) bandage comprising:
   a membrane configured for disposition over a wound so as to form a wound chamber between the membrane and the wound, the membrane comprising a wound-side surface, an atmosphere-side surface, and an opening extending from the wound-side surface to the atmosphere-side surface, the opening being in fluid communication with the wound chamber;
   wherein the membrane comprises a transparent layer and an absorbent layer, wherein the absorbent layer is disposed on the wound-side of the membrane;
   wherein the absorbent layer comprises a plurality of windows extending through the absorbent layer; and
   further wherein the transparent layer of the membrane and the plurality of windows of the absorbent layer of the membrane permit visualization of the wound through the membrane; and
   a pump assembly carried by the membrane, the pump assembly comprising:
      a pump body comprising a wall structure defining a pump chamber, wherein at least a portion of the wall structure is resilient;
      a wound-side passageway extending through the wall structure and communicating with the wound chamber through the opening formed in the membrane; and
      an atmosphere-side passageway extending through the wall structure and connecting the pump chamber to the atmosphere;
      wherein the pump assembly is configured to generate a negative air pressure within the wound chamber;
positioning the NPWT bandage over the wound so as to form a wound chamber between the membrane and the wound;
generating a negative air pressure within the wound chamber; and
visualizing the absorbent layer of the membrane to determine whether the NPWT bandage needs to be removed from the wound and replaced with a new NPWT bandage.

For illustration purposes only, a method for covering a wound releasing exudate is described, the method comprising:
providing a bandage comprising:
   a membrane configured for disposition over a wound so as to form a wound chamber between the membrane and the wound, the membrane comprising a wound-side surface, an atmosphere-side surface, and an opening extending from the wound-side surface to the atmosphere-side surface, the opening being in fluid communication with the wound chamber;
   wherein the membrane comprises a transparent layer and an absorbent layer, wherein the absorbent layer is disposed on the wound-side of the membrane;
   wherein the absorbent layer comprises a plurality of windows extending through the absorbent layer; and
   further wherein the transparent layer of the membrane and the plurality of windows of the absorbent layer of the membrane permit visualization of the wound through the membrane;
positioning the bandage over the wound so as to form a wound chamber between the membrane and the wound; and
visualizing the absorbent layer of the membrane to determine whether the bandage needs to be removed from the wound and replaced with a new bandage.

### Brief Description Of The Drawings

These and other objects and features of the present invention will be more fully disclosed or rendered obvious by the following detailed description, which is to be considered together with the accompanying drawings wherein like numbers refer to like parts, and further wherein:
Figs. 1-4 are schematic views showing a NPWT bandage for reference purposes only, with Figs. 2 and 3 being exploded views;

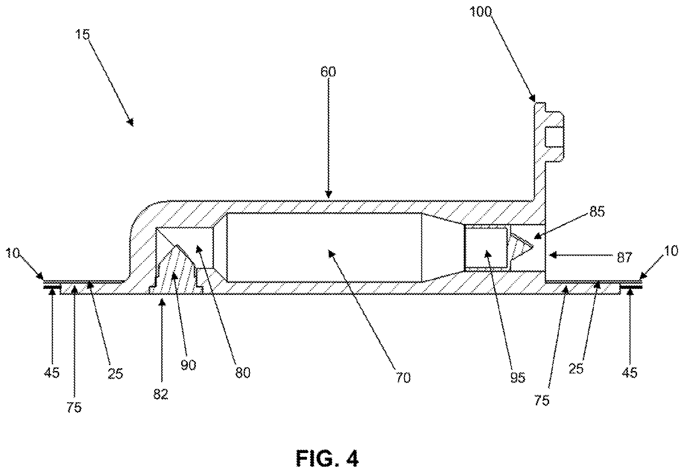

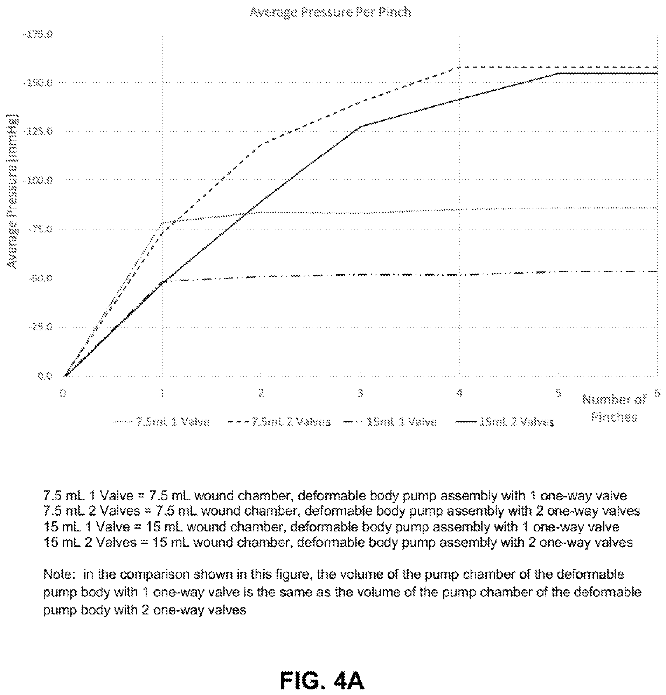

Fig. 4A is a schematic view showing, for two different size wound chambers (i.e., a 7.5 mL wound chamber and a 15 mL wound chamber), a maximum negative pressure that may be established with (i) a deformable pump body having two one-way valves (with one one-way valve being disposed on either side of the deformable pump body), and (ii) a deformable pump body having a single one-way valve (note: in the comparison shown in Fig. 4A, the volume of the pump chamber of the deformable pump body with 1 one-way valve is the same as the volume of the pump chamber of the deformable pump body with 2 one-way valves);
Figs. 5 and 6 are schematic views showing the pump body of the pump assembly of the NPWT bandage shown in Figs. 1-4 in its substantially fully expanded configuration (Fig. 5) and in its substantially fully collapsed configuration (Fig. 6);
Fig. 7 is a schematic view showing how the pump body of the pump assembly of the NPWT bandage shown in Figs. 1-4 abruptly changes state between its substantially fully expanded configuration and its substantially fully collapsed configuration;
Fig. 8 is a schematic view showing how the pump bodies of the pump assemblies of prior art NPWT bandages gradually change state between their substantially fully expanded configuration and their substantially fully collapsed configuration;
Figs. 9-16 are schematic views showing exemplary use of the NPWT bandage shown in Figs. 1-4 (note that in Figs. 11 and 14-16, removable cap 100 (see below) is removed from the figures for clarity of illustration);
Figs. 17-20 are schematic views showing another NPWT bandage for reference purposes only;
Fig. 20A is a schematic view showing how the configuration of the pump body changes during use (note that in Fig. 20A, only the outer boundary of the pump's side wall 65 is shown, and the pump's flange 75 and neck 77 are omitted, for clarity of illustration);
Figs. 21-25 are schematic views showing another NPWT bandage for reference purposes only;
Figs. 26 and 27 are schematic views showing another NPWT bandage for reference purposes only, wherein the pump body is constructed so that it also provides a negative pressure decay "warning state" indicator;
Fig. 28 is a schematic view showing how the configuration of the pump body comprising a negative pressure decay "warning state" indicator changes during use (note that in Fig. 28, only the outer boundary of the pump's side wall is shown, for clarity of illustration);
Fig. 29 is a schematic view showing a new and improved NPWT bandage formed in accordance with the present invention;
Fig. 30 is a schematic view showing another new and improved NPWT bandage formed in accordance with the present invention;
Fig. 31 is a schematic view showing another new and improved NPWT bandage formed in accordance with the present invention;
Fig. 32 is a schematic view showing another NPWT bandage for reference purposes only;
Figs. 33 and 34 are schematic views showing another NPWT bandage for reference purposes only;
Figs. 35-38 are schematic views showing another NPWT bandage for reference purposes only, wherein the wound chamber comprises a lateral extension, and wherein the pump assembly is in fluid communication with the lateral extension, so that the pump assembly is laterally offset from the wound while remaining in fluid communication with the wound;
Figs. 39-42 are schematic views showing another NPWT bandage for reference purposes only, wherein the wound chamber comprises a lateral extension, and wherein the pump assembly is in fluid communication with the lateral extension, so that the pump assembly is laterally offset from the wound while remaining in fluid communication with the wound;
Figs. 43-46 are schematic views showing another NPWT bandage for reference purposes only, wherein the wound chamber comprises a lateral extension, and wherein the pump assembly is in fluid communication with the lateral extension, so that the pump assembly is laterally offset from the wound while remaining in fluid communication with the wound;
Fig. 46A is a schematic view showing another NPWT bandage for reference purposes only, wherein the wound chamber comprises a lateral extension, and wherein the pump assembly is in fluid communication with the lateral extension, so that the pump assembly is laterally offset from the wound while remaining in fluid communication with the wound;

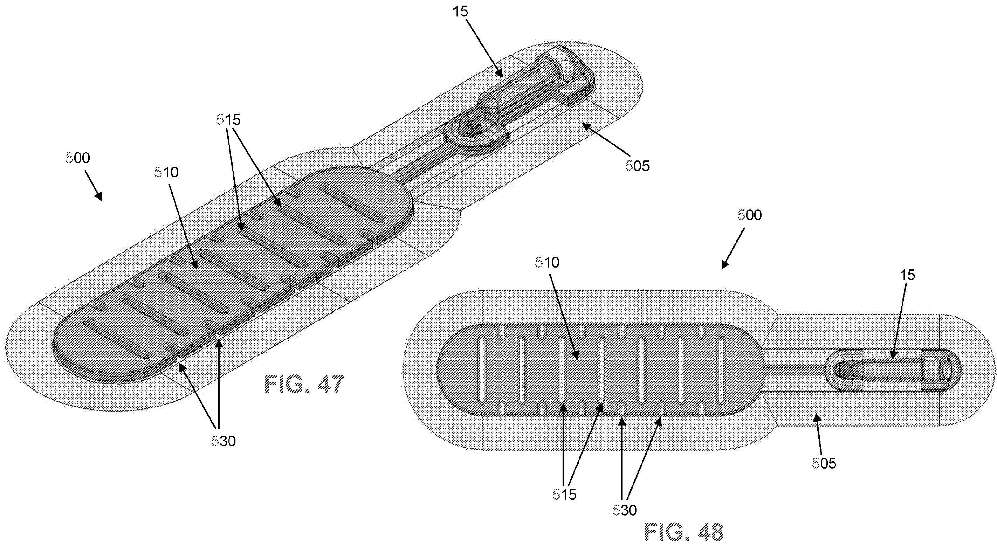

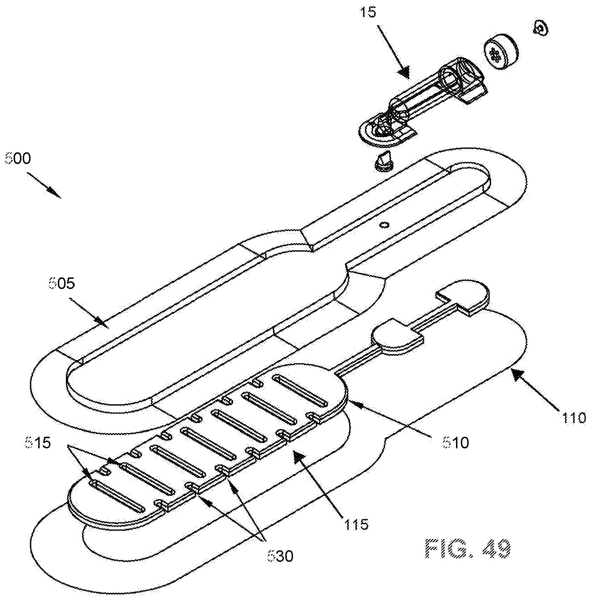

Figs. 47, 48 and 49 are schematic views showing another NPWT bandage for reference purposes only, wherein the bandage comprises a transparent top layer which captures a layer of foam against the wound, wherein the foam layer is provided with a series of windows which (i) allow direct visualization of the wound, and (ii) provide a metric along the length of the bandage to indicate whether the bandage needs to be replaced, and further wherein the foam layer also comprises perimeter serrations which allow the bandage to conform to a curved wound - in this form, the NPWT bandage comprises an on-board pump which is laterally offset from the wound;
Fig. 50 is a schematic view showing further details of the foam layer of Figs. 47, 48 and 49;
Figs. 51 and 52 are schematic views showing various levels of exudate in the foam layer of the bandage of Fig. 47;
Figs. 53 and 54 are schematic views showing how the windows in the foam layer allow medical personnel to align the bandage along a wound line;
Fig. 55 is a schematic view showing perimeter serrations formed in the foam layer which allow the bandage to be curved so as to follow the path of a curved wound;
Figs. 56 and 57 are schematic views showing how the NPWT bandage of Figs. 47, 48 and 49 may be modified so as to position the on-board pump over a portion of the foam; and

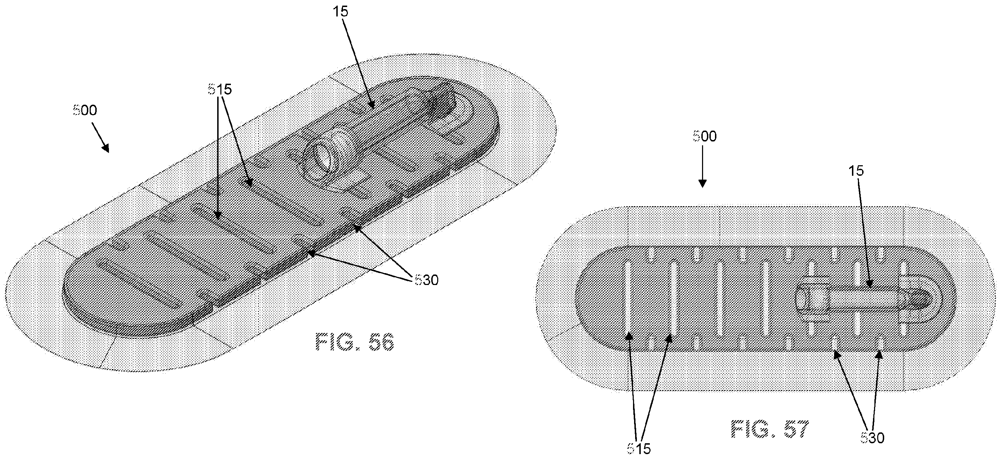

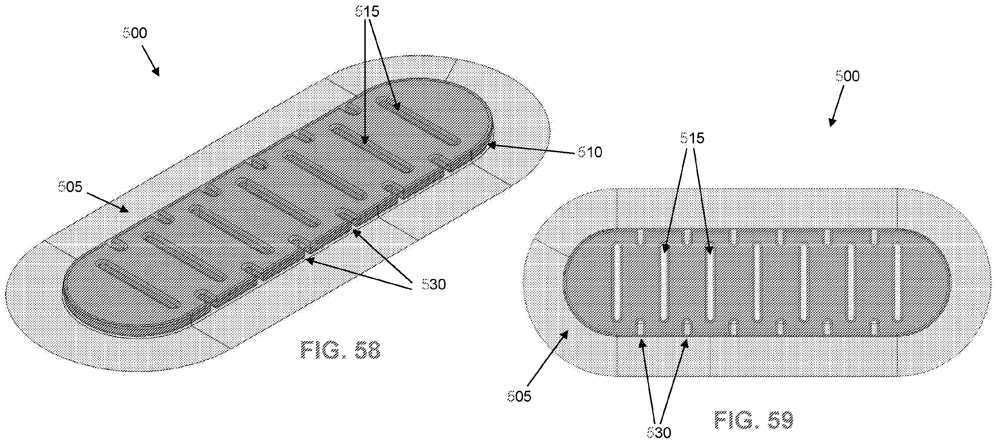

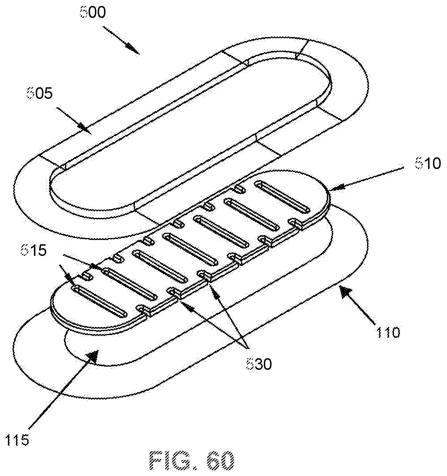

Figs. 58, 59 and 60 are schematic views showing how the NPWT bandage of Figs. 56 and 57 may be modified to remove the on-board pump, whereby to provide a general purpose bandage which comprises a transparent top layer which captures a layer of foam against the wound, wherein the foam layer is provided with a series of windows which (i) allow direct visualization of the wound, and (ii) provide a metric along the length of the bandage to indicate whether the bandage needs to be replaced, and further wherein the foam layer also comprises perimeter serrations which allow the bandage to conform to a curved wound.

### Detailed Description Of The Preferred Embodiments

The present invention comprises the provision of a new and improved NPWT bandage which is simple, inexpensive, easy-to-use, small in size (including having a low profile), is atraumatic to the wound during use, has improved pump efficiency, incorporates an automatic pressure indicator for indicating the level of negative pressure created, and provides an automatic pressure limiter for limiting the level of negative pressure created.

### The Manually-Operated Negative Pressure Wound Therapy (NPWT) Bandage In General

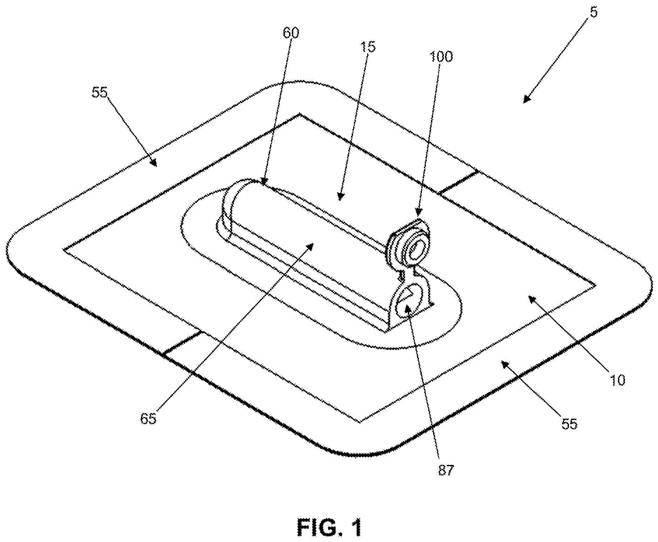

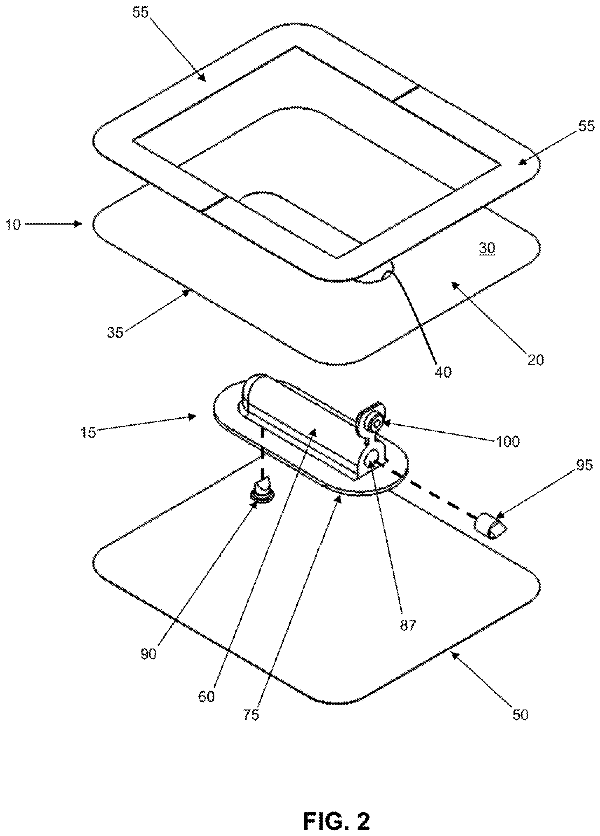

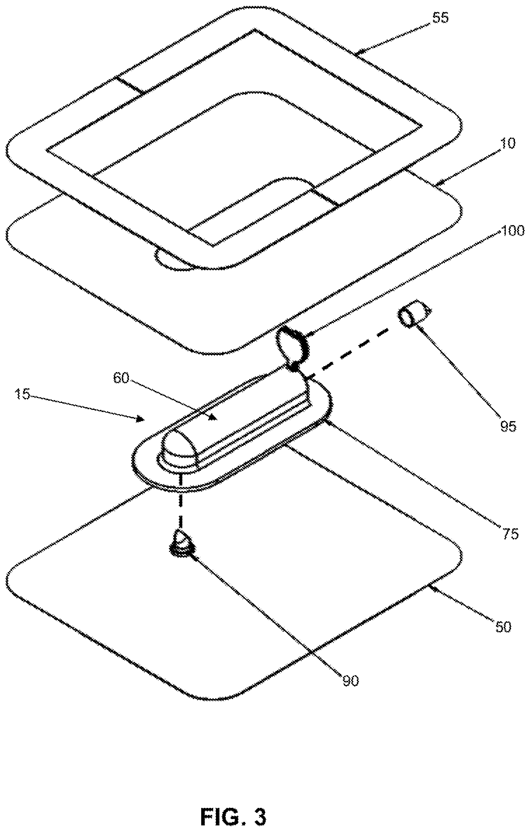

More particularly, and looking first at Figs. 1-4, there is shown a manually-operated negative pressure wound therapy (NPWT) bandage 5 having improved pump efficiency, an automatic pressure indicator for indicating the level of negative pressure created, and an automatic pressure limiter for limiting the level of negative pressure created.

NPWT bandage 5 generally comprises a membrane (or sheet) 10 and a pump assembly 15.

As will hereinafter be discussed, membrane 10 is configured to make a fully-sealed chamber around the perimeter of a wound, whereby to define a wound chamber.

And as will hereinafter be discussed, pump assembly 15 is configured to apply a negative pressure to the fully-sealed wound chamber, such that any contaminants and microbes present at the wound site are drawn away from the wound, exudates are drawn out of the wound, and beneficial biological responses are promoted at the wound site. Significantly, pump assembly 15 is designed to provide improved pump efficiency, an automatic pressure indicator for indicating the level of negative pressure created, and an automatic pressure limiter for limiting the level of negative pressure created, as will hereinafter be discussed.

### The Membrane

More particularly, membrane 10 comprises a flat planar sheet 20 formed out of a flexible, substantially air-impermeable material, e.g., Tegaderm from 3M Company (which has also been known as the Minnesota Mining and Manufacturing Company), so that it can conform to body contours and form a substantially air-tight chamber around the perimeter of a wound (i.e., the wound chamber). Membrane 10 is characterized by a wound-side surface 25 and an atmosphere-side surface 30. Membrane 10 is also characterized by an outer perimeter 35 and an inner opening 40.

An adhesive 45 is preferably disposed on wound-side surface 25 of membrane 10. A release liner 50 is preferably disposed on wound-side surface 25 atop adhesive 45 so as to keep adhesive 45 covered until use.

A removable stiffener 55 is preferably disposed on atmosphere-side surface 30 of membrane 10. Removable stiffener 55 serves to facilitate manipulation of NPWT bandage 5 (and particularly membrane 10) during removal of the NPWT bandage from its sterile packaging and during positioning of the NPWT bandage about a wound. Removable stiffener 55 is intended to be removed from membrane 10 once NPWT bandage 5 has been secured about the wound site. Removable stiffener 55 may be provided as a single element or, more preferably, removable stiffener 55 is provided as a pair of elements so as to facilitate removal from membrane 10 after NPWT bandage 5 has been secured about the wound site.

### The Pump Assembly

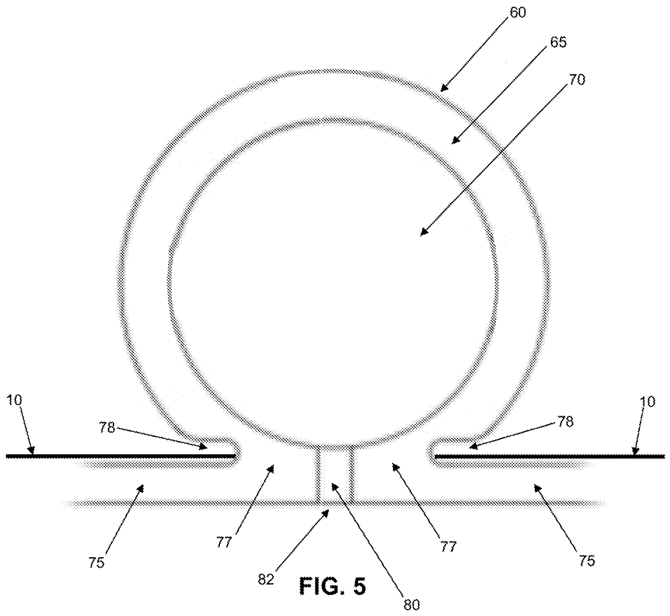

Pump assembly 15 comprises a pump body 60 having a generally cylindrical shape and comprising a side wall 65 and an inner chamber 70. Pump body 60 is formed out of a resilient material, e.g., silicone, such that side wall 65 may be compressed inwardly by the application of an external force (e.g., squeezing by the thumb and forefinger of a user) and will then attempt to return to its original uncompressed state when the external force is removed. A pump flange 75 is preferably formed on one side of pump body 60. As will hereinafter be discussed in further detail, pump body 60 extends through inner opening 40 of membrane 10, and the upper surface of pump flange 75 is secured to the wound-side surface 25 of membrane 10 so that pump assembly 15 is secured to, and carried by, membrane 10. Pump flange 75 is preferably formed out of a flexible material so that it can conform (to at least a limited extent) to body contours. In one form, pump body 60 and pump flange 75 are formed integral with one another out of the same material, e.g., silicone. In one preferred form, side wall 65 of pump body 60 and pump flange 75 merge at a neck 77 (Fig. 5). And in one preferred form, neck 77 has a relatively small width relative to the full diameter of pump body 60, with recesses 78 extending inwardly between membrane 10 and pump body 60, such that pump body 60 is mounted to pump flange 75 but is still free to radially compress/radially expand with minimal interference from pump flange 75. A wound-side passageway 80 is formed in pump body 60 and communicates with inner chamber 70. Wound-side passageway 80 opens on the exterior of pump body 60 at a wound-side port 82. An atmosphere-side passageway 85 is formed in pump body 60 and also communicates with inner chamber 70. Atmosphere-side passageway 85 opens on the exterior of pump body 60 at an atmosphere-side port 87.

A wound-side one-way valve 90 is disposed in wound-side passageway 80 and is configured to permit fluid to enter inner chamber 70 through wound-side passageway 80 but to prevent fluid from exiting inner chamber 70 through wound-side passageway 80.

An atmosphere-side one-way valve 95 is disposed in atmosphere-side passageway 85 and is configured to permit fluid to exit inner chamber 70 through atmosphere-side passageway 85 but to prevent fluid from entering inner chamber 70 through atmosphere-side passageway 85.

As a result of this construction, when pump body 60 of pump assembly 15 is manually squeezed (e.g., by applying a compressive force to side wall 65 of pump body 60 with the thumb and forefinger of a user), fluid (e.g., air, liquid, etc.) within inner chamber 70 will be forced out of inner chamber 70 via atmosphere-side passageway 85, and when pump body 60 of pump assembly 15 is thereafter released (e.g., by relaxing the compressive force applied to side wall 65 of pump body 60 by the thumb and forefinger of a user), fluid (e.g., air, liquid, etc.) below wound-side surface 25 of membrane 10 (e.g., air, liquid, etc. within the wound chamber) will be drawn into inner chamber 70 through wound-side passageway 80 as the resilient side wall of the pump body returns to its uncompressed state.

Note that when pump body 60 of pump assembly 15 is manually squeezed, fluid (e.g., air, liquid, etc.) within inner chamber 70 is prevented from exiting inner chamber 70 through wound-side passageway 80 due to the one-way operation of wound-side one-way valve 90, and when pump body 60 of pump assembly 15 is thereafter released, air from the atmosphere is prevented from being drawn into inner chamber 70 through atmosphere-side passageway 85 due to the one-way operation of atmosphere-side one-way valve 95.

Thus it will be appreciated that repeatedly manually squeezing and releasing pump body 60 of pump assembly 15 will apply suction to the wound chamber disposed below wound-side surface 25 of membrane 10, whereby to create negative pressure at the wound site.

It should be appreciated that the approach of providing a pump assembly utilizing two one-way valves disposed on either side of a deformable pump body with an in-line configuration (i.e., wound-side one-way valve 90 and atmosphere-side one-way valve 95 disposed on either side of deformable pump body 60) provides a number of significant advantages which are not achievable with the prior art's approach of providing a deformable pump body utilizing a single one-way valve.

More particularly, and as will hereinafter be discussed, the approach of providing a pump assembly utilizing two one-way valves disposed on either side of a deformable pump body with an in-line configuration (i.e., wound-side one-way valve 90 and atmosphere-side one-way valve 95 disposed on either side of deformable pump body 60) allows substantially the same maximum negative pressure to be established at the wound site regardless of the size of the wound chamber. This is not achievable with the prior art's approach of providing a deformable pump body utilizing a single one-way valve.

In addition, the approach of providing a pump assembly utilizing two one-way valves disposed on either side of a deformable pump body with an in-line configuration (i.e., wound-side one-way valve 90 and atmosphere-side one-way valve 95 disposed on either side of deformable pump body 60) allows a greater constant selected maximum negative pressure to be achieved at the wound site than can be achieved at the wound site using a deformable pump body with a single one-way valve (which is reflective of the prior art's approach).

More particularly, Fig. 4A, shows, for two different size wound chambers (i.e., a 7.5 mL wound chamber and a 15 mL wound chamber), a maximum negative pressure that may be established with (i) a deformable pump body having two one-way valves (with one one-way valve being disposed on either side of the deformable pump body), and (ii) a deformable pump body having a single one-way valve (note: in the comparison shown in Fig. 4A, the volume of the pump chamber of the deformable pump body with 1 one-way valve is the same as the volume of the pump chamber of the deformable pump body with 2 one-way valves).

First, Fig. 4A shows that using a deformable pump body having two one-way valves (with one one-way valve being disposed on either side of the deformable pump body) to evacuate the wound chamber lets you establish substantially the same maximum negative pressure in the wound chamber regardless of the size of the wound chamber (i.e., it yields approximately -150.0 mm Hg for a 7.5 mL wound chamber and approximately -150.0 mm Hg for a 15 mL wound chamber), whereas using a deformable pump body having a single one-way valve does not (i.e., it yields approximately -80.0 mm Hg for a 7.5 mL wound chamber and approximately -50.0 mm Hg for a 15 mL wound chamber). Thus, the NPWT bandage allows substantially the same maximum negative pressure to be established at the wound site regardless of the size of the wound chamber, whereas prior art NPWT bandages do not.

This unique feature is clinically significant, inasmuch as (i) it is generally desirable to establish a selected maximum negative pressure at the wound site (e.g., between about 60 mm Hg and about 180 mm Hg), and (ii) it is generally difficult to know in advance the volume of the wound chamber (e.g., due to variations in medical applications, variations in patient anatomy, etc.). Thus, inasmuch as the NPWT bandage as described allows substantially the same maximum negative pressure to be established at the wound site regardless of the size of the wound chamber, the present disclosure allows the NPWT bandage to be engineered in advance (e.g., at the time of manufacture) to establish a selected maximum negative pressure at the wound site, whereas prior art NPWT bandages do not.

Second, Fig. 4A shows that using a deformable pump body having two one-way valves (with one one-way valve being disposed on either side of the deformable pump body) to evacuate the wound chamber lets you establish a substantially higher maximum negative pressure in the wound chamber (i.e., it yields approximately -150.0 mm Hg for a 7.5 mL wound chamber and approximately -150.0 mm Hg for a 15 mL wound chamber) than can be established using a deformable pump body having a single one-way valve (i.e., it yields approximately -80.0 mm Hg for a 7.5 mL wound chamber and approximately -50.0 mm Hg for a 15 mL wound chamber). Thus, the NPWT bandage as disclosed allows a substantially higher maximum negative pressure to be established at the wound site.

Note also that the pressure within inner chamber 70 of pump body 60 is generally equal to the pressure below wound-side surface 25 of membrane 10 (i.e., the pressure within inner chamber 70 of pump body 60 is generally equal to the pressure within the wound chamber).

In one preferred form, pump assembly 15 also comprises a removable cap 100. Removable cap 100 is configured to selectively close off atmosphere-side passageway 85 to fluid flow when removable cap 100 is inserted into atmosphere-side passageway 85 so as to close off atmosphere-side port 87.

Pump assembly 15 is mounted to membrane 10 such that pump assembly 15 is carried by membrane 10. More particularly, pump assembly 15 is mounted to membrane 10 by (i) passing pump body 60 of pump assembly 15 through inner opening 40 of membrane 10, (ii) bringing pump flange 75 up against wound-side surface 25 of membrane 10, and then (iii) adhering pump flange 75 to wound-side surface 25 of membrane 10 (e.g., by bonding, gluing, etc.). Note that pump assembly 15 and membrane 10 make a substantially air-tight connection.

Significantly, pump body 60 of pump assembly 15 is carefully configured to provide (i) improved pump efficiency, (ii) an automatic pressure indicator for indicating the level of negative pressure created, and (iii) an automatic pressure limiter for limiting the level of negative pressure created, as will hereinafter be discussed.

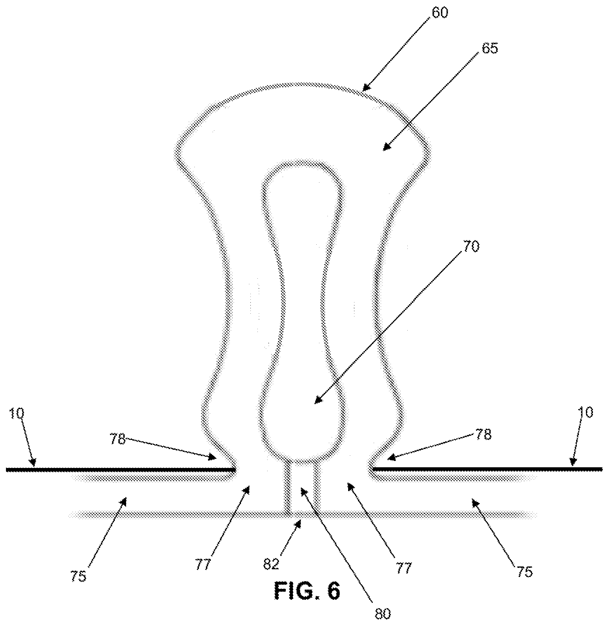

More particularly, pump body 60 of pump assembly 15 is specifically configured so that the pump body will abruptly change state between (i) a substantially fully expanded configuration where side wall 65 of pump body 60 and inner chamber 70 of pump body 60 have a substantially circular cross-section (see Fig. 5) when the pressure differential between the pressure of the fluid within inner chamber 70 and atmospheric pressure is below a given threshold, and (ii) a substantially fully collapsed configuration where side wall 65 of pump body 60 bows inwardly (see Fig. 6) when the pressure differential between the pressure of the fluid within inner chamber 70 and atmospheric pressure exceeds a given threshold.

Specifically, when the pressure differential between the pressure of the fluid within inner chamber 70 and atmospheric pressure is below a given threshold, pump body 60 of pump assembly 15 will assume its substantially fully expanded configuration (Fig. 5), and when the pressure differential between the pressure of the fluid within inner chamber 70 and atmospheric pressure is above a given threshold, pump body 60 of pump assembly 15 will assume its substantially fully collapsed configuration (Fig. 6).

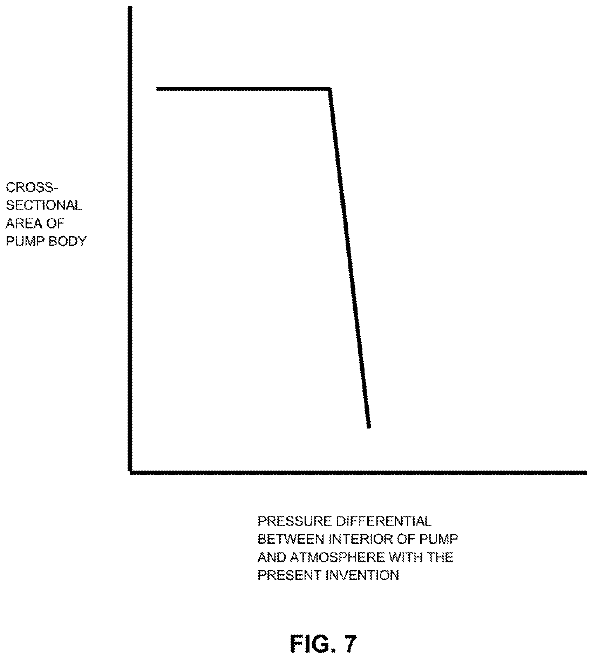

Significantly, pump body 60 of pump assembly 15 is configured so that it will abruptly change state between its substantially fully expanded configuration (Fig. 5) and its substantially fully collapsed configuration (Fig. 6) when the pressure differential between the pressure of the fluid within inner chamber 70 and atmospheric pressure crosses the aforementioned given threshold. See Fig. 7, which is a graph showing the relationship between the diameter of side wall 65 of pump body 60 and the pressure differential between the pressure of a fluid within inner chamber 70 and atmospheric pressure. Thus, pump assembly 15 is specifically configured to essentially behave as a substantially "binary state" device - it is either substantially fully expanded (Fig. 5) or substantially fully collapsed (Fig. 6). In this respect it should be appreciated that, as used herein, the term "substantially "binary state" device" is intended to refer to a device which is inclined to assume either a substantially fully expanded condition or a substantially fully collapsed condition and, as used herein, the term "substantially "binary state" behavior" is intended to refer to the inclination of a device to assume either a substantially fully expanded condition or a substantially fully collapsed condition.

Note that the substantially "binary state" behavior of pump body 60 is a consequence of forming the pump body with a side wall 65 having a substantially circular cross-section, which gives the pump body an "over-the-center" deformation characteristic, i.e., the side wall of pump body 60 has a "failure" mode where it abruptly transitions from its substantially fully expanded configuration to its substantially fully collapsed configuration, and has a "restoration" mode where it abruptly transitions from its substantially fully collapsed configuration to its substantially fully expanded configuration. See Fig. 7. Note that by forming pump assembly 15 so that side wall 65 of pump body 60 and pump flange 75 merge at a neck 77 (Fig. 5), with neck 77 having a relatively small width relative to the full diameter of pump body 60, and with recesses 78 extending inwardly between membrane 10 and pump body 60, pump body 60 has a substantially circular cross-section over substantially its entire circumference, with pump body 60 free to radially compress/radially expand with minimal interference from pump flange 75, so that pump body 60 can exhibit substantially "binary state" behavior.

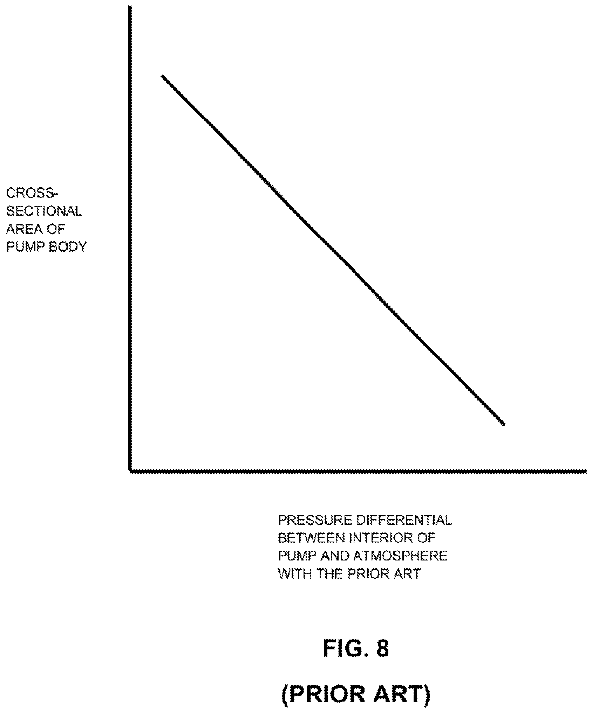

Note also that the prior art approaches of forming the pump body with dome-like or square pump configurations does not provide the pump body with an abrupt change of state - rather, these prior art dome-like or square pump configurations provide the pump body with a more gradual change of state between an expanded configuration and a collapsed configuration when the pressure differential between the pressure of the fluid within an inner chamber and atmospheric pressure changes. See Fig. 8, which is a graph showing the relationship between the diameter of the side wall of a pump body having a dome-like or square configuration and the pressure differential between the pressure of a fluid within an inner chamber of the pump body and atmospheric pressure.

As a result of deliberately configuring side wall 65 of pump body 60 of pump assembly 15 to exhibit this abrupt change of state, pump assembly 15 is able to provide improved pump efficiency, an automatic pressure indicator for indicating the level of negative pressure created, and an automatic pressure limiter for limiting the level of negative pressure created.

More particularly, by configuring pump body 60 of pump assembly 15 so that it will abruptly change state between its substantially fully expanded configuration and its substantially fully collapsed configuration when the pressure differential between the pressure of a fluid within inner chamber 70 and atmospheric pressure crosses a given threshold, pump assembly 15 effectively returns to its substantially fully expanded configuration as long as the pressure differential between the pressure of the fluid within inner chamber 70 and atmospheric pressure is below the given threshold. As a result, so long as the pressure differential between the fluid within inner chamber 70 and atmospheric pressure is below the given threshold, pump assembly 15 returns to its substantially fully expanded configuration between compressions (i.e., squeezes), and hence remains fully efficient as it applies a negative pressure to the wound chamber. This is in contrast to the performance of prior art devices where the pump body exhibits a gradual change of state between an expanded configuration and a collapsed configuration when the pressure differential between the pressure of a fluid within the inner chamber of the pump assembly changes, which makes the pump assembly progressively less efficient as it reduces the pressure within the wound chamber. This is because the pump body will progressively return less and less to its fully expanded configuration as negative pressure is created in the wound chamber, so that the pump assembly is able to evacuate less and less fluid with each squeeze of the pump body. In other words, with prior art devices, the pump assembly becomes less and less efficient as negative pressure is created in the wound chamber.

In a related manner, by configuring pump body 60 so that it will abruptly change state between its substantially fully expanded configuration and its substantially fully collapsed configuration when the pressure differential between the pressure of the fluid within inner chamber 70 and atmospheric pressure crosses a given threshold, pump assembly 15 is able to function as an automatic pressure indicator for indicating the level of negative pressure created, i.e., so long as pump body 60 of pump assembly 15 returns to its substantially fully expanded configuration between squeezes, it will be readily apparent to an observer that the pressure within inner chamber 70 (and hence the pressure within the wound chamber) will be less than a given level. This is in marked contrast to the performance of prior art devices where pump body 60 provides a gradual change of state between an expanded configuration and a collapsed configuration when the pressure differential between the pressure of a fluid within an inner chamber of the pump assembly changes, in which case the pump assembly is not able to function as an automatic pressure indicator for indicating the level of negative pressure created.

And also in a related manner, by configuring pump body 60 so that it will abruptly change state between its substantially fully expanded configuration and its substantially fully collapsed configuration when the pressure differential between the pressure of a fluid within inner chamber 70 and atmospheric pressure crosses a given threshold, pump assembly 15 is able to function as an automatic pressure limiter for limiting the level of negative pressure created since, as soon as pump body 60 assumes its substantially fully collapsed configuration, pump assembly 15 is no longer able to pump fluid from the wound chamber, essentially deactivating the pump assembly. This is in marked contrast to the performance of prior art devices where the pump body provides a gradual change of state between an expanded configuration and a collapsed configuration when the pressure differential between the pressure of a fluid within an inner chamber changes, since the pump assembly is not effectively deactivated at a given pressure differential.

It should be appreciated that the pressure differential required to transition pump body 60 between its substantially fully-expanded configuration and its substantially fully-collapsed configuration (i.e., the aforementioned "given threshold") may be "tuned" (i.e., tailored) to a particular level by varying one or more characteristics of pump body 60, e.g., by forming side wall 65 of pump body 60 out of a material having a particular durometer, by adjusting the thickness of side wall 65 of pump body 60, by adjusting the diameter of inner chamber 70 of pump body 60, etc.

In general, it has been found that excellent therapeutic results may be achieved when the pressure differential required to transition pump body 60 between its substantially fully-expanded configuration and its substantially fully-collapsed configuration (i.e., the aforementioned "given threshold") is between about 60 mm Hg and about 180 mm Hg. In other words, it has been found that excellent therapeutic results may be achieved where pump body 60 transitions between its substantially fully-expanded configuration (Fig. 5) and its substantially fully-collapsed configuration (Fig. 6) at a negative pressure of between about 60 mm Hg and about 180 mm Hg. It is believed that where pump body 60 transitions between its two states at a lower pressure (i.e., where pump body 60 transitions at a negative pressure lower than about 60 mm Hg), not enough suction is provided at the wound site to effectively draw contaminants and microbes away from the wound site and/or to effectively draw exudates away from the wound site and/or to promote beneficial biological responses at the wound site. It is also believed that where pump body 60 transitions between its two states at a higher pressure (i.e., where pump body 60 transitions at a negative pressure higher than about 180 mm Hg), the suction provided at the wound site may cause trauma to the tissue (e.g., blistering, capillary leakage, etc.).

In one preferred form, pump body 60 of pump assembly 15 is configured so that it abruptly transitions between its substantially fully expanded configuration and its substantially fully collapsed configuration when the pressure differential between the pressure of the fluid within inner chamber 70 and atmospheric pressure exceeds 80 mm Hg. Thus, in this form, as long as the negative pressure within the wound chamber is less than 680 mm Hg (assuming atmospheric pressure is 760 mm Hg), pump assembly 15 returns to its substantially fully expanded configuration between squeezes of the pump body and maintains its pump efficiency as it applies suction to the wound chamber, and as soon as the negative pressure within the wound chamber exceeds 680 mm Hg (assuming atmospheric pressure is 760 mm Hg), pump assembly 15 will assume its substantially fully collapsed configuration, acting as an automatic pressure indicator to indicate that the level of negative pressure created at the wound site has exceeded 80 mm Hg and automatically deactivating pump assembly 15 so that the level of negative pressure created at the wound site cannot exceed 80 mm Hg.

Note that inasmuch as pump body 60 of pump assembly 15 has a substantially cylindrical configuration, NPWT bandage 5 has a low profile.

Note also that inasmuch as pump body 60 of pump assembly 15 is configured to be squeezed between the thumb and forefinger of a user, the compressive force being applied to pump body 60 is applied parallel to the surface of the skin, so that no trauma is applied to the wound during use (i.e., during pumping of pump assembly 15). This is in marked contrast to prior art NPWT bandages which employ a dome-like configuration and require the compressive force to be applied toward the wound.

### Exemplary Use

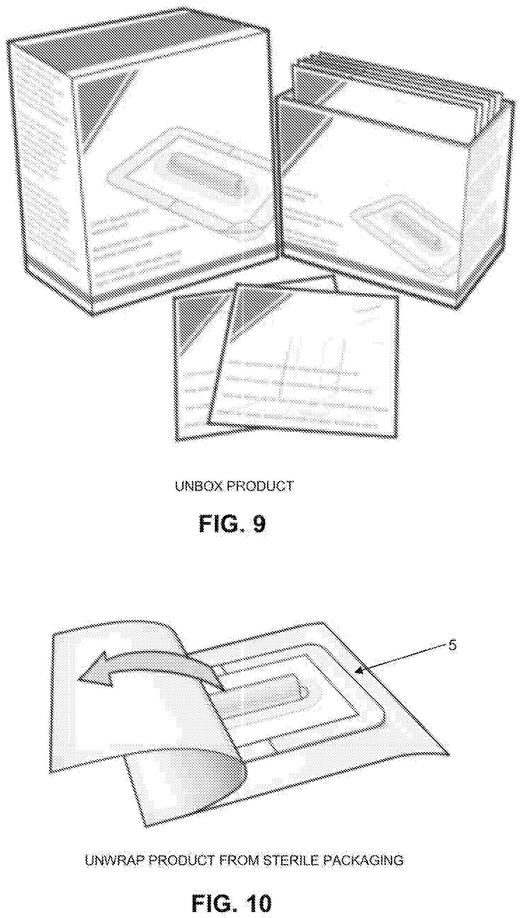

Looking now at Figs. 9-16, NPWT bandage 5 is intended to be used as follows.

First, an NPWT bandage 5 is removed from its box. In one form each individual NPWT bandage 5 is contained in a separate sterile package, with multiple sterile packages contained in a box. See Fig. 9.

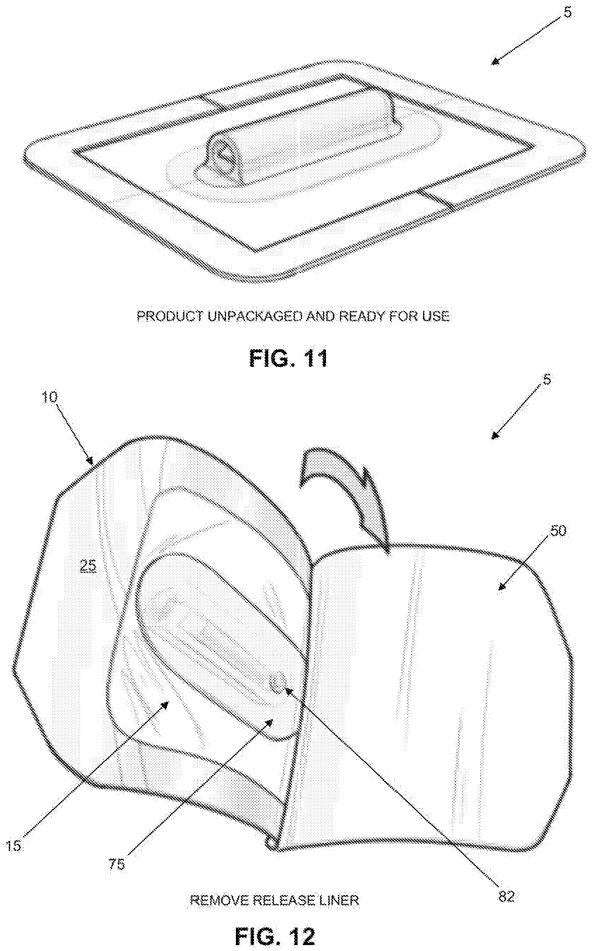

Next, an NPWT bandage 5 is removed from its sterile package (Fig. 10) so as to be ready for use (Fig. 11).

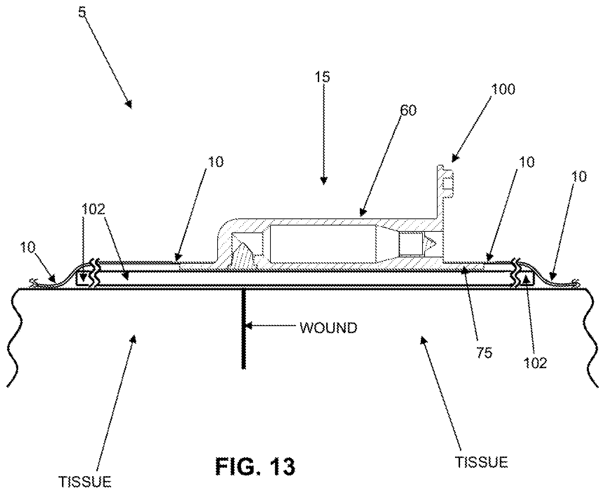

In order to apply NPWT bandage 5 to the wound site, release liner 50 is removed from wound-side surface 25 of membrane 10. See Fig. 12. Then NPWT bandage 5 is positioned against the skin of a patient so that wound-side surface 25 of membrane 10 is positioned against the wound, with adhesive 45 securing NPWT bandage 5 to the skin of the patient, thereby forming a substantially air-tight seal with the skin of the patient about the perimeter of the wound chamber. See Fig. 13.

Note that when NPWT bandage 5 is applied to the skin of the patient, wound-side port 82 of wound-side passageway 80 of pump assembly 15 is open to the wound chamber.

Note also that a layer of gauze (or other absorbent wound dressing) 102 may be placed on the wound site prior to placing NPWT bandage 5 on the skin of the patient, so that the layer of gauze (or other absorbent wound dressing) is interposed between the wound and wound-side passageway 80 of pump assembly 15. As a result, exudate emerging from the wound will be taken up by the gauze (or other absorbent wound dressing). Note that, if desired, the layer of gauze (or other absorbent wound dressing) 102 may be mounted to (i.e., secured to) the wound-side surface of membrane 10, e.g., such as at the time of manufacture, so that the layer of gauze (or other absorbent wound dressing) 102 is carried to the wound site by NPWT bandage 5 and is applied to the wound at the same time as the NPWT bandage 5.

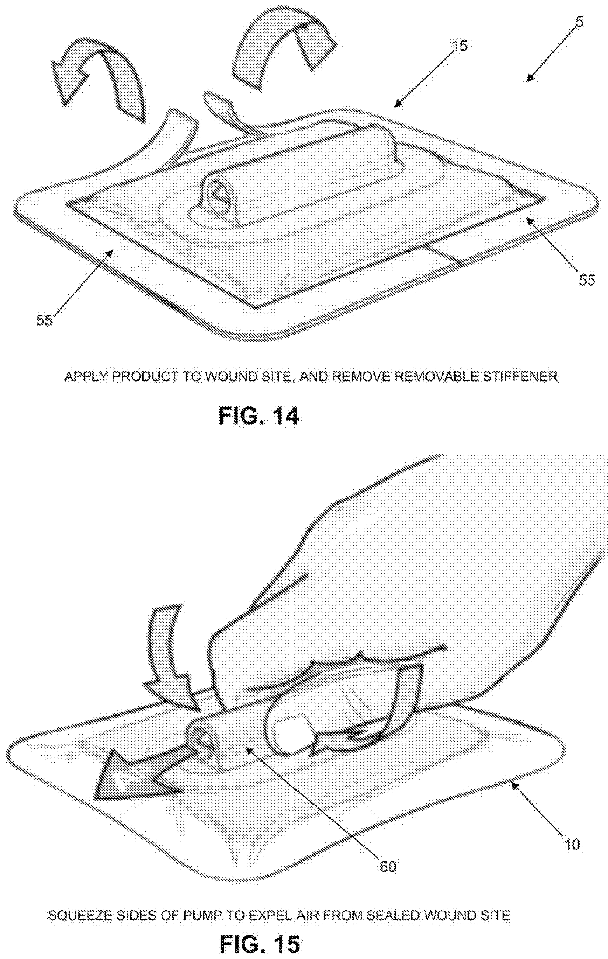

Next, with NPWT bandage 5 secured to the skin of the patient, removable stiffener 55 is removed from atmosphere-side surface 30 of membrane 10. See Fig. 14.

At this point, NPWT bandage 5 may be used to apply negative pressure to the wound chamber. This is done by squeezing side wall 65 of pump body 60 between the thumb and forefinger of a user so as to compress pump body 60 into its substantially fully collapsed configuration, whereby to expel fluid (e.g., air, liquid, etc.) from inner chamber 70 of pump body 60 via atmosphere-side passageway 85 and atmosphere-side one-way valve 95. See Fig. 15. Note that fluid in inner chamber 70 of pump body 60 is prevented from exiting inner chamber 70 through wound-side passageway 80 due to the presence of wound-side one-way valve 90. Then side wall 65 of pump body 60 is released, allowing the resilient pump body 60 to return to its substantially fully expanded configuration, thereby creating a negative pressure within inner chamber 70 and wound-side passageway 80, such that fluid below wound-side surface 25 of membrane 10 (e.g., fluid within the wound chamber) is drawn into inner chamber 70 through wound-side passageway 80 and wound-side one-way valve 90. Note that air in the atmosphere is prevented from entering inner chamber 70 through atmosphere-side passageway 85 due to the presence of atmosphere-side one-way valve 95.

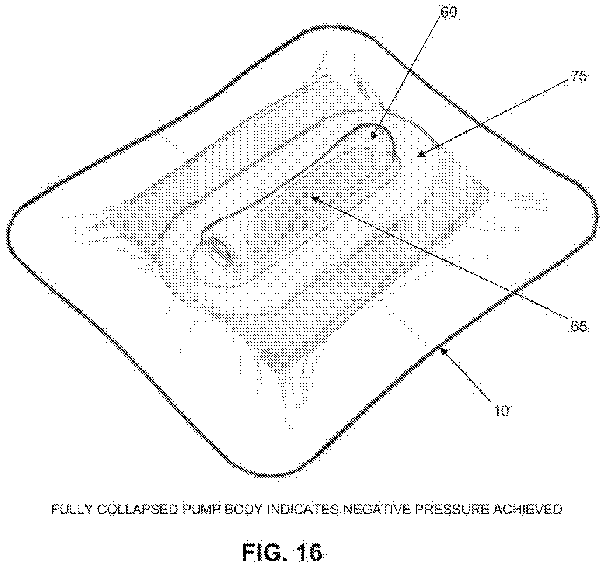

This process of squeezing and releasing side wall 65 of pump body 60 is repeated until pump body 60 of pump assembly 15 remains in its substantially fully collapsed configuration (i.e., side wall 65 of pump body 60 bows inwardly) even when side wall 65 of pump body 60 is not being manually compressed. See Fig. 16. When pump body 60 of pump assembly 15 remains in its substantially fully collapsed configuration even when side wall 65 of pump body 60 is not being manually compressed, an observer will know that the pressure differential between the pressure of the fluid within inner chamber 70 (and the wound chamber) and atmospheric pressure exceeds the desired threshold, indicating that the desired level of negative pressure has been achieved at the wound site. Note that when pump body 60 of pump assembly 15 remains in its substantially fully collapsed configuration even when side wall 65 of pump body 60 is not being manually compressed, pump assembly 15 will have been effectively deactivated, since it will be impossible to continue using the pump assembly with side wall 65 in its substantially fully collapsed configuration.

At this point removable cap 100 may be used to seal atmosphere-side port 87 of atmosphere-side passageway 85.

NPWT bandage 5 is left in place on the wound for an appropriate period of time (e.g., a few days) so as to shield the wound from contaminants and microbes during healing, draw exudates out of the wound, and promote beneficial biological responses at the wound site. In the event that leakage should cause the negative pressure created in the wound chamber to fall below the given threshold (which will be apparent to an observer by virtue of the fact that side wall 65 of pump body 60 will return to its substantially fully-expanded configuration), atmosphere-side port 87 of atmosphere-side passageway 85 may be unsealed (i.e., by removing removable cap 100) and then pump assembly 15 may be used in the manner discussed above to re-establish the desired negative pressure in the wound chamber (i.e., by repeatedly squeezing and releasing side wall 65 of pump body 60).

When appropriate, NPWT bandage 5 may be removed from the skin of the patient by simply peeling membrane 10 away from the skin of the patient.

### Pump Body With Notches To Enhance The Substantially "Binary State" Behavior Of The Pump Body

As noted above, pump body 60 of pump assembly 15 is preferably specifically configured so that the pump body will abruptly change state between (i) a substantially fully expanded configuration where side wall 65 of pump body 60 and inner chamber 70 of pump body 60 have a substantially circular cross-section (see Fig. 5) when the pressure differential between the pressure of the fluid within inner chamber 70 and atmospheric pressure is below a given threshold, and (ii) a substantially fully collapsed configuration where side wall 65 of pump body 60 bows inwardly (see Fig. 6) when the pressure differential between the pressure of the fluid within inner chamber 70 and atmospheric pressure exceeds a given threshold.

As also noted above, this substantially "binary state" behavior of pump body 60 is achieved by forming the pump body with a substantially circular cross-section, which gives the body an "over-the-center" deformation characteristic, i.e., so that the side wall of pump body 60 has a "failure" mode where it abruptly transitions from a substantially fully expanded configuration to a substantially fully collapsed configuration, and has a "restoration" mode where it abruptly transitions from a substantially fully collapsed configuration to a substantially fully expanded configuration. See Fig. 7. As noted above, by forming pump assembly 15 so that side wall 65 of pump body 60 and pump flange 75 merge at a neck 77 (Fig. 5), with neck 77 having a relatively small width relative to the full diameter of pump body 60, and with recesses 78 extending inwardly between membrane 10 and pump body 60, pump body 60 has a substantially circular cross-section over substantially its entire circumference, with pump body 60 free to radially compress/radially expand with minimal interference from pump flange 75, so that pump body 60 can exhibit substantially "binary state" behavior.

If desired, pump body 60 can be modified so as to enhance the substantially "binary state" behavior of the pump body.

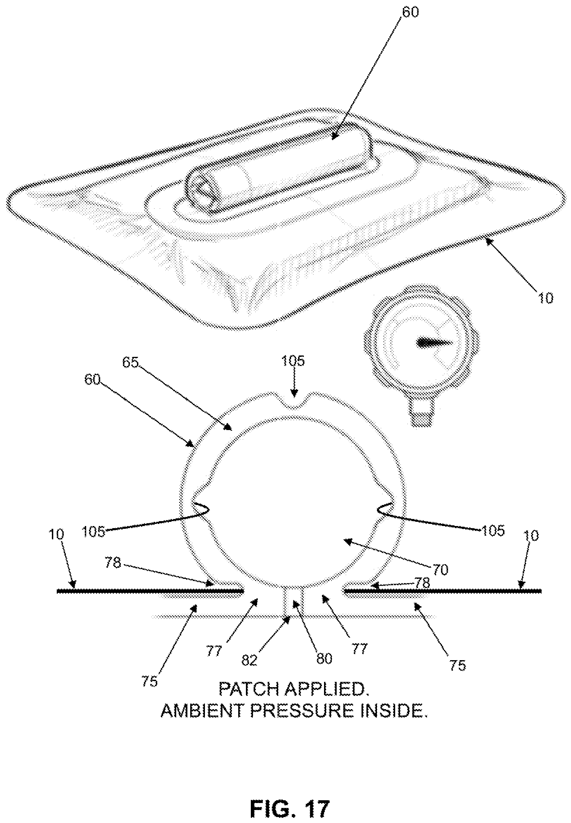

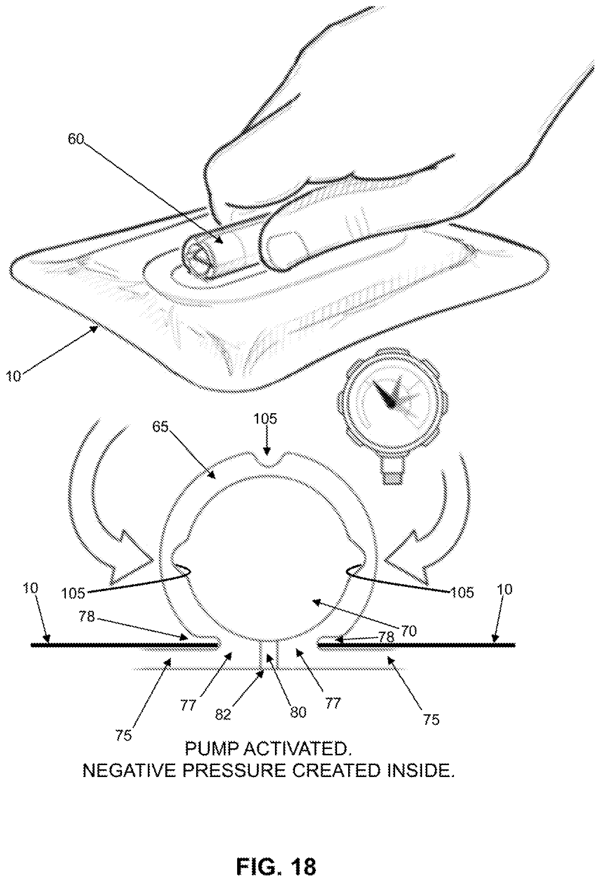

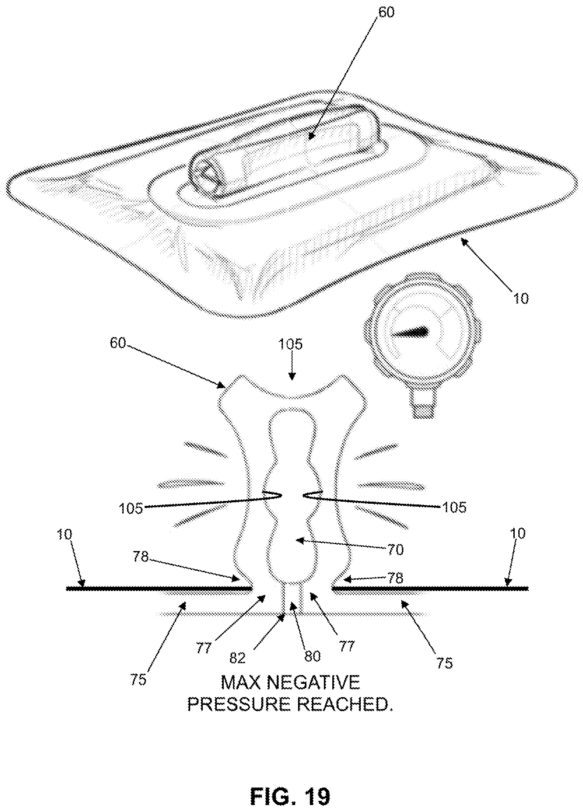

By way of example but not limitation, and looking now at Figs. 17-20, notches 105 can be formed in pump body 60 (e.g., at the "9 o'clock", "12 o'clock" and "3 o'clock" positions) so as to enhance the substantially "binary state" behavior of the pump body by further inducing pump body 60 to assume only its substantially fully expanded configuration or its substantially fully collapsed configuration. Note that the more that pump body 60 exhibits true "binary state" behavior, the more that pump efficiency will improve and the better that pump assembly 15 will serve as an automatic pressure indicator and as an automatic pressure limiter.

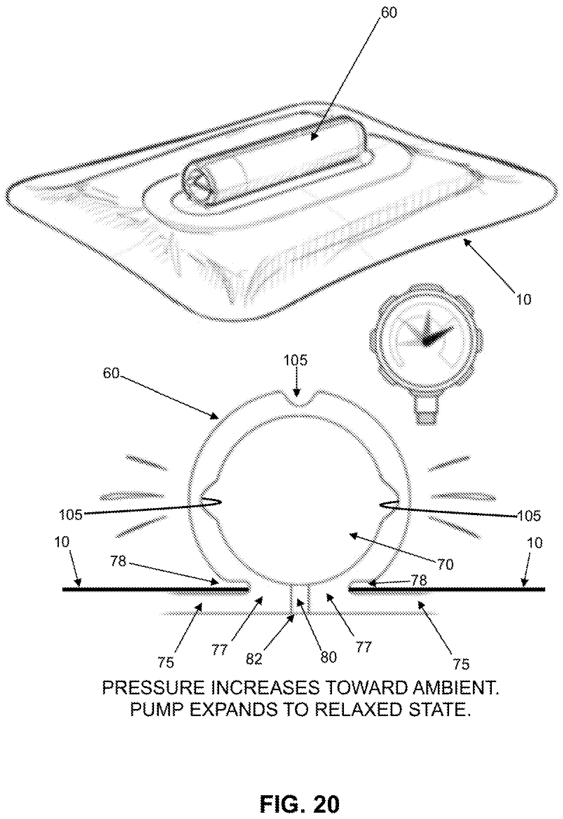

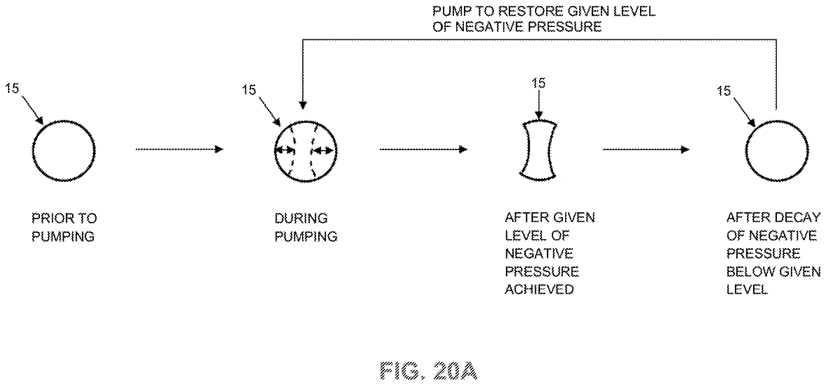

Thus it will be seen that, and looking now at Fig. 20A, pump body 60 generally has:
a substantially fully expanded configuration prior to pumping;
a collapsing and expanding configuration during pumping;
a "vertically-oriented" substantially collapsed configuration after a given level of negative pressure has been achieved by pumping; and
a substantially fully expanded configuration after the negative pressure has decayed below a given level.

Furthermore, the desired negative pressure can be re-established after transition to the substantially fully expanded configuration by renewed pumping.

### NPWT Bandage Incorporating Gauze (Or Other Absorbent Wound Dressing) And Utilizing An Improved Pump Assembly

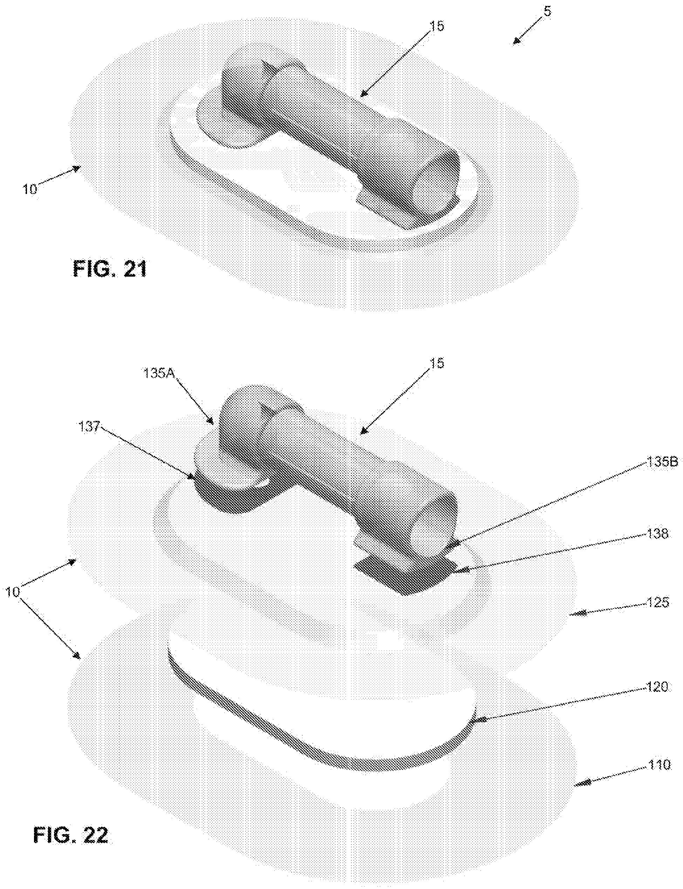

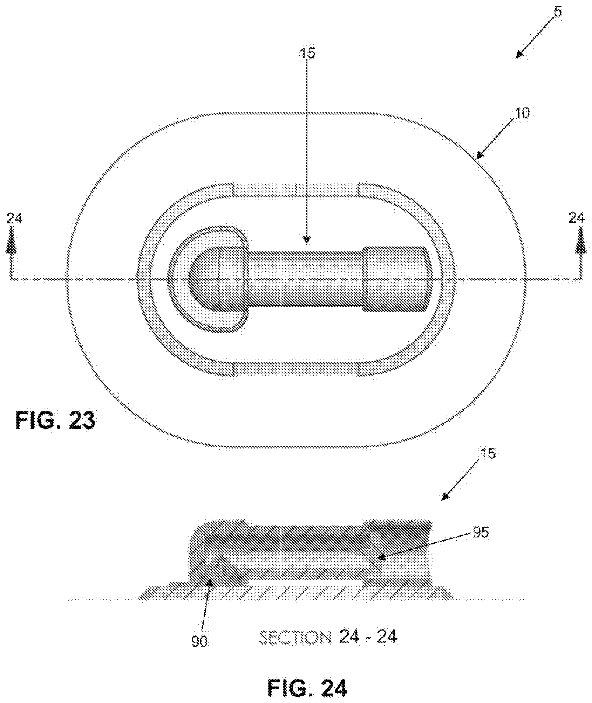

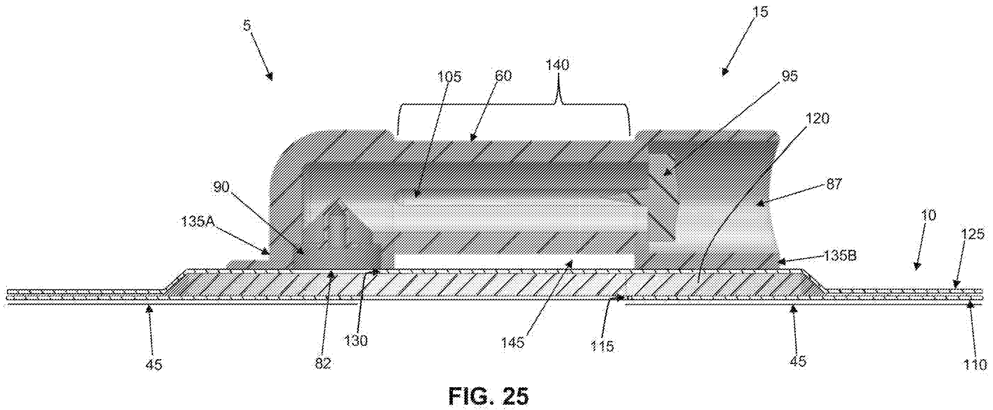

Looking next at Figs. 21-25, there is shown another negative pressure wound therapy (NPWT) bandage 5. The NPWT bandage 5 shown in Figs. 21-25 is substantially the same as the NPWT bandage 5 shown in Figs. 1-16, and the NPWT bandage 5 shown in Figs. 17-20, except that (i) in the construction shown in Figs. 21-25, membrane 10 comprises multiple layers which incorporate gauze (or other absorbent wound dressing), and (ii) in the construction shown in Figs. 21-25, pump assembly 15 has a modified construction and is secured to membrane 10 using a different approach.

More particularly, in this form, membrane 10 comprises a lower skin-contacting polyurethane layer 110 having a center opening 115, an intermediate foam (or gauze or other absorbent wound dressing) layer 120 for disposition over center opening 115 of lower skin-contacting polyurethane layer 110, and an upper polyurethane layer 125 for disposition over intermediate foam layer 120 and lower skin-contacting polyurethane layer 110. In the preferred form, upper polyurethane layer 125 is formed out of a substantially air-impermeable material. And, in the preferred form, upper polyurethane layer 125 and lower skin-contacting polyurethane layer 110 have the same size outer perimeter, so that upper polyurethane layer 125 does not contact the skin of the patient. The outer perimeters of upper polyurethane layer 125 and lower skin-contacting polyurethane layer 110 are secured to one another, capturing intermediate foam layer 120 therebetween. Intermediate foam layer 120 has an outer perimeter which is (i) larger than the perimeter of center opening 115 of lower skin-contacting polyurethane layer 110, and (ii) smaller than the outer perimeter of the outer perimeters of lower skin-contacting polyurethane layer 110 and upper polyurethane layer 125. In this way, when NPWT bandage 5 has its center opening 115 of lower skin-contacting polyurethane layer 110 positioned over a wound, fluid from the wound can pass through center opening 115 of lower skin-contacting polyurethane layer 110 to reach intermediate foam layer 120. It will be appreciated that adhesive 45 is positioned on the wound side surface of lower skin-contacting polyurethane layer 110 so that a substantially air-tight seal may be established by NPWT bandage 5 about the perimeter of a wound (i.e., so as to form the aforementioned wound chamber). An opening 130 is formed in upper polyurethane layer 125, and overlaps center opening 115 of lower skin-contacting polyurethane layer 110, so that wound-side passageway 80 of pump assembly 15 can access fluid (e.g., air, liquid, etc.) within the wound chamber (i.e., via opening 130 in upper polyurethane layer 125, the openings in intermediate foam layer 120, and center opening 115 of lower skin-contacting polyurethane layer 110) for evacuation during pumping of pump assembly 15.

The pump assembly 15 utilized in the NPWT bandage 5 of Figs. 21-25 is generally similar to the pump assembly 15 described above, except that it comprises a pair of pedestals 135A, 135B for mounting pump assembly 15 to membrane 10. More particularly, pedestal 135A comprises one end of pump body 60 and is adhered (e.g., by an adhesive 137) to the upper surface of membrane 10 (i.e., to the upper surface of upper polyurethane layer 125) so that wound-side passageway 80 and wound-side one-way valve 90 are aligned with opening 130 in upper polyurethane layer 125 (and hence in fluid communication with the wound chamber). Pedestal 135B comprises the other end of pump body 60 and is adhered (e.g., by an adhesive 138) to the upper surface of membrane 10 (i.e., to the upper surface of upper polyurethane layer 125). The intervening portion 140 of pump body 60 sits suspended between pedestal 135A and pedestal 135B, elevated above upper polyurethane layer 125 of membrane 10, so that a space 145 is formed between intervening portion 140 of pump body 60 and upper polyurethane layer 135 of membrane 10. Inasmuch as intervening portion 140 of pump body 60 is not mounted directly to membrane 10, but is instead suspended above membrane 10 by means of pedestals 135A and 135B, intervening portion 140 of pump body 60 can be formed with a true circular cross-section, whereby to enhance the substantially "binary state" behavior of the NPWT bandage. It will be appreciated that pump body 60 may incorporate one or more of the aforementioned notches 105 so as to further enhance the substantially "binary state" behavior of the NPWT bandage.

### NPWT Bandage With Negative Pressure Decay "Warning State" Indicator

It has also been discovered that, if desired, pump body 60 can be configured to provide a negative pressure decay "warning state" indicator.

More particularly, by controlling the number, configuration and disposition of notches 105, pump body 60 can provide a "warning state" indicator to indicate that a decay of the negative pressure has occurred, albeit not enough of a decay to cause the binary state transition of the pump body (i.e., from a substantially collapsed configuration to a substantially fully expanded configuration). By way of example but not limitation, by providing a pump body having notches 105 which are appropriate in number, configuration and disposition, the pump body can provide a "warning state" indicator (to indicate that there is a decay of the negative pressure below a given level, albeit not enough of a decay to cause a binary state transition of the pump body from substantially fully collapsed to substantially fully expanded) by transitioning the pump body from its "vertically-oriented" substantially collapsed configuration (which occurs after a given level of negative pressure has been achieved) to a "horizontally-oriented" substantially collapsed configuration (which occurs after there has been some decay of the negative pressure, albeit not enough of a decay to cause a binary state transition of the pump body back to a substantially fully expanded configuration).

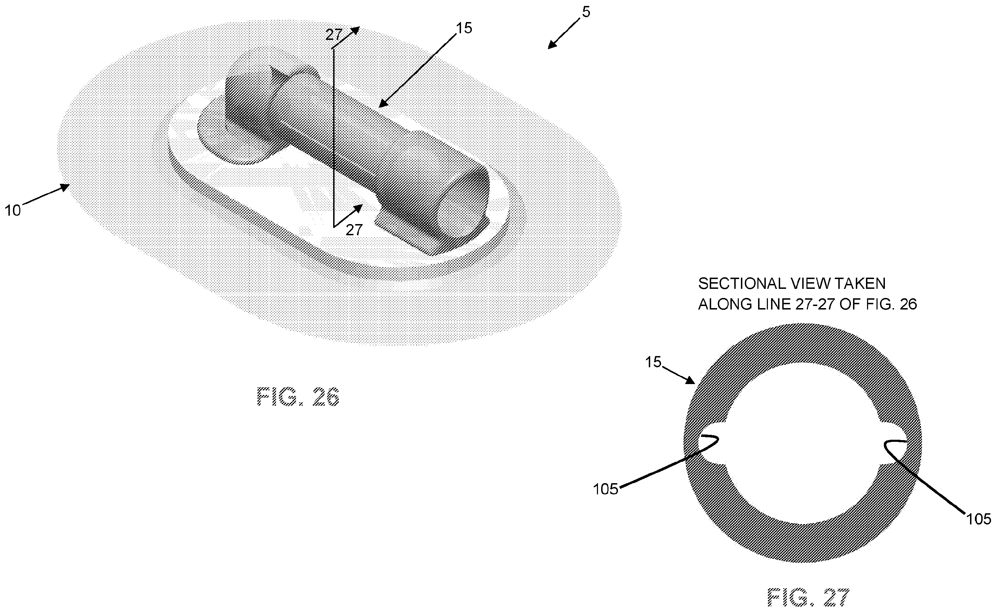

Figs. 26 and 27 show an exemplary construction wherein a pump body, having notches 105 which are appropriate in number, configuration and disposition, can provide a "warning state" indicator (to indicate that there is a decay of the negative pressure below a given level, albeit not enough of a decay to cause a binary state transition of the pump body to a fully expanded configuration) by transitioning the pump body from its "vertically-oriented" substantially collapsed configuration (which occurs after a given level of negative pressure has been achieved) to a "horizontally-oriented" substantially collapsed configuration (which occurs after there has been some decay of the negative pressure, albeit not enough of a decay to cause a binary state transition of the pump body back to a substantially fully expanded configuration).

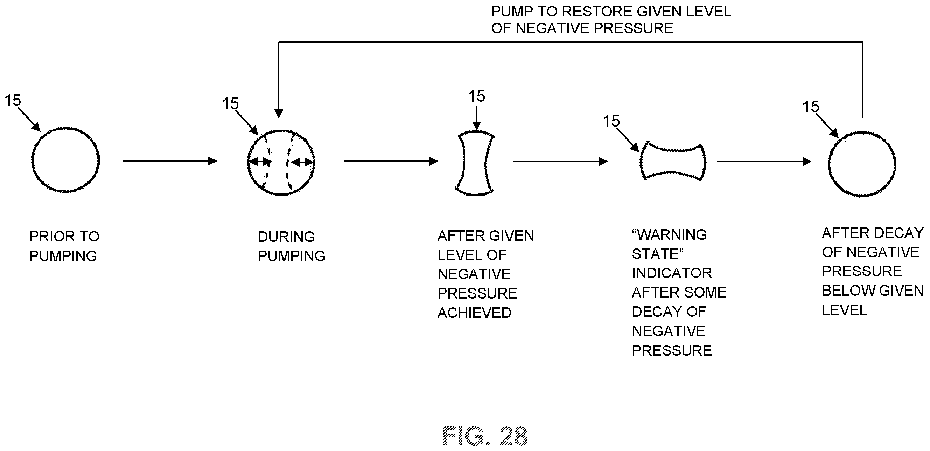

Thus it will be seen that, looking now at Fig. 28, pump body 60 generally has:
a substantially fully expanded configuration prior to pumping;
a collapsing and expanding configuration during pumping;
a "vertically-oriented" substantially collapsed configuration after a given level of negative pressure has been achieved by pumping;
a "horizontally-oriented" substantially collapsed configuration after a given level of negative pressure has been achieved but there is a decay of the negative pressure, albeit not enough of a decay to cause a binary state transition of the pump body to a substantially fully expanded configuration; and
a substantially fully expanded configuration after the negative pressure has decayed below a given level.

Furthermore, the desired negative pressure can be re-established after transition to the substantially fully expanded configuration by renewed pumping.

### NPWT Bandage With Canister For Collecting Exudates

In some situations a wound may produce significant levels of exudates. In these situations, it may be desirable for the negative pressure wound therapy (NPWT) bandage to include a canister for collecting the exudates.

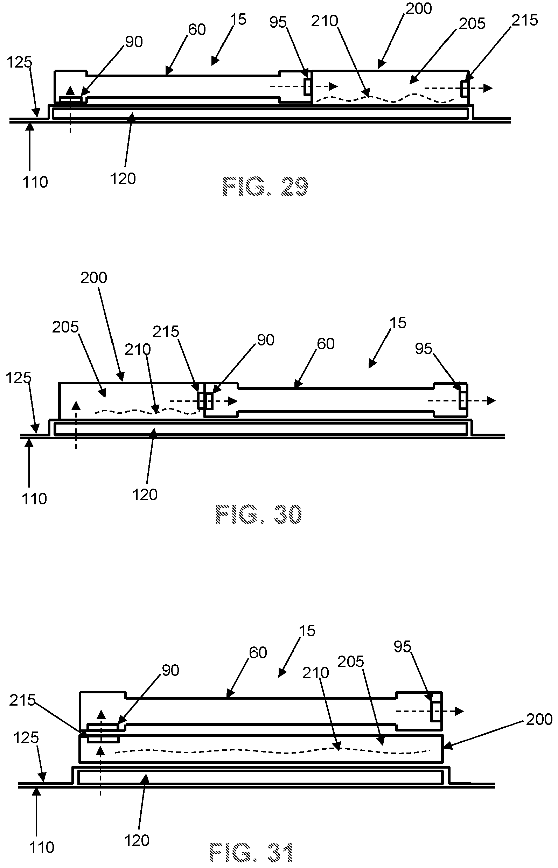

Thus, for example, and looking now at Fig. 29, there is shown a negative pressure wound therapy (NPWT) bandage 5 which includes a canister 200 for collecting exudates. In this form of the invention, atmosphere-side one-way valve 95 of pump assembly 15 outputs to the interior 205 of canister 200 so that exudates 210 collect in canister 200. In one form of the invention, canister 200 includes an opening which is covered by a membrane 215, wherein membrane 215 is gas permeable, but not fluid permeable, such that gases may escape from canister 200 but liquids will be trapped within canister 200.

In another form of the invention, and looking now at Fig. 30, canister 200 may be disposed between the wound chamber and wound-side one-way valve 90 of pump assembly 15. More particularly, in this form of the invention, interior 205 of canister 200 is in fluid communication with the wound chamber and is in fluid communication with wound-side one-way valve 90 of pump assembly 15, such that pumping pump assembly 15 will create a negative pressure within canister 200 and hence within the wound chamber. Preferably canister 200 includes the aforementioned membrane 215 (which is gas permeable but not liquid permeable) adjacent to wound-side one-way valve 90 of pump assembly 15, such that gases may escape from canister 200, but liquids will be trapped within canister 200 and not pass into pump assembly 15.

In still another form of the invention, and looking now at Fig. 31, canister 200 is disposed between the wound chamber and wound-side one-way valve 90 of pump assembly 15, in a manner similar to the construction of Fig. 30, except that pump assembly 15 is mounted atop canister 200.

It should be appreciated that canister 200 may be formed integral with pump body 60, or it may be formed separately from pump body 60 and simply be fluidly connected to pump body 60, but in any case canister 200 is preferably mounted to some portion of negative pressure wound therapy (NPWT) bandage 5 so that canister 200 moves in conjunction with the remainder of the bandage.

Furthermore, if desired, canister 200 may contain (or have one or more of its internal surfaces coated with) an absorbent or superabsorbent material to absorb liquid exudates evacuated from the wound chamber. By way of example but not limitation, canister 200 may contain (or have one or more of its internal surfaces coated with) absorbent or superabsorbent crystals or hydrocolloids or gel-forming matter.

Canister 200 may be used with any of the pump assemblies disclosed herein. By way of example but not limitation, canister 200 may be used with pump assembly 15 shown in Figs. 1-4, pump assembly 15 shown in Figs. 17-20A, pump assembly 15 shown in Figs. 21-25, pump assembly 15 shown in Figs. 26-28, etc.

### NPWT Bandage With Luer Lock For Suction Attachment

In some situations it may be desirable to make negative pressure wound therapy (NPWT) bandage 5 in a large size, so that it can cover a large wound chamber. This can be accomplished by increasing the size of membrane (or sheet) 10. However, it may not be desirable to increase the size of pump assembly 15, since it is typically desirable for the pump assembly to maintain a low, non-intrusive profile. But increasing the size of membrane (or sheet) 10 while maintaining the size of pump assembly 15 can be clinically undesirable, since then many more pump compressions may be required in order to evacuate the large wound chamber to the correct negative pressure level. Stated another way, increasing the size of membrane (or sheet) 10 while maintaining the size of pump assembly 15 has the effect of increasing the number of pump compressions required to establish the correct negative pressure level in the wound chamber. Thus, clinical considerations for the size of the pump assembly, and clinical considerations for the number of pump compressions to be used to establish the correct negative pressure level in the wound chamber, may limit, in a practical sense, the size of membrane (or sheet) 10.

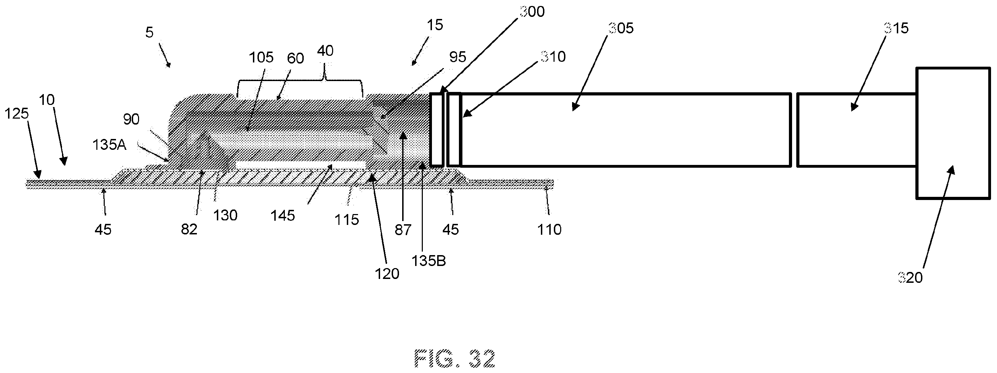

In order to address this, negative pressure wound therapy (NPWT) bandage 5 may be configured for connection to a source of suction, such that a suction source can be applied to NPWT bandage 5 so as to establish the correct negative pressure level in the wound chamber without requiring compression of pump body 60. By way of example but not limitation, and looking now at Fig. 32, NPWT bandage 5 may comprise one half 300 of a luer lock, and a suction line 305 may comprise the second half 310 of a luer lock, such that suction line 305 can be fluidly connected to atmosphere-side port 87 of pump body 60. When suction line 305 has its other end 315 connected to a functioning source of suction 320, suction source 320 can apply suction to pump body 60. This suction is then automatically applied to the wound chamber, such that suction source 320 can establish negative pressure in the wound chamber.

Significantly, when the correct level of negative pressure is established in the wound chamber, the walls of pump body 60 will collapse in the manner previously described. Thus, even where suction source 320 is used to create negative pressure in the wound chamber, NPWT bandage 5 still acts as a visual indicator of when the proper level of negative pressure has been established in the wound chamber, since pump assembly 15 transitions from its substantially fully expanded configuration to its substantially fully collapsed configuration when the proper level of negative pressure has been established in the wound chamber.

After the proper level of negative pressure has been established in the wound chamber using suction source 320, suction line 305 can then be disconnected from NPWT bandage 5.

Thereafter, if the negative pressure level in the wound chamber should decay, pump assembly 15 can be used to re-establish the correct negative pressure level in the wound chamber, either by compressing pump body 60 or by reconnecting NPWT bandage 5 to suction source 320.

**In** the preferred form discussed above, NPWT bandage 5 comprises one half of a luer lock, and suction line 305 comprises the second half of a luer lock. However, the disclosure is not restricted to the use of a luer lock connection - other types of fluid connectors may be used to connect suction line 305 to pump assembly 15.

As discussed above, NPWT bandage 5 serves as a visual indicator of when the proper level of negative pressure has been established in the wound chamber, i.e., by transitioning from its substantially fully expanded configuration to its substantially fully collapsed configuration.

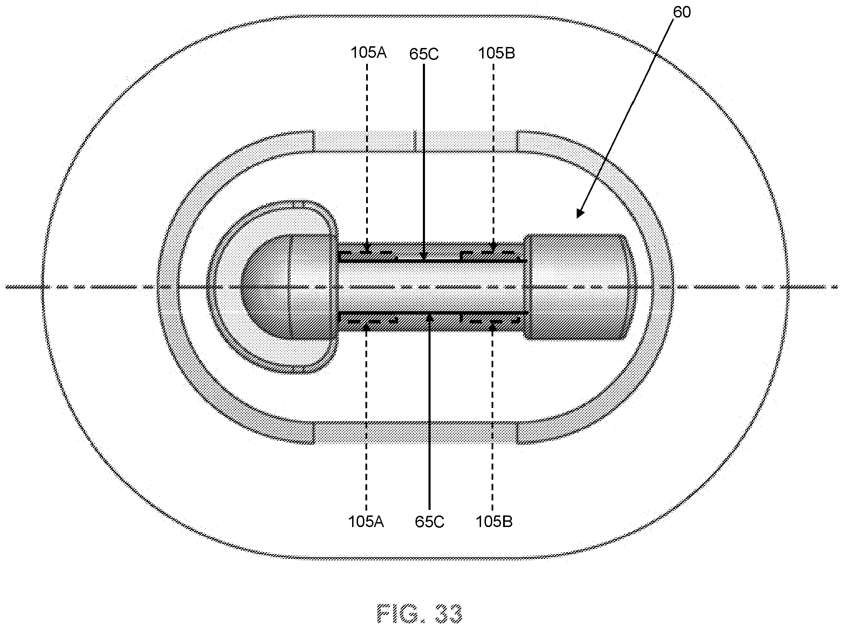

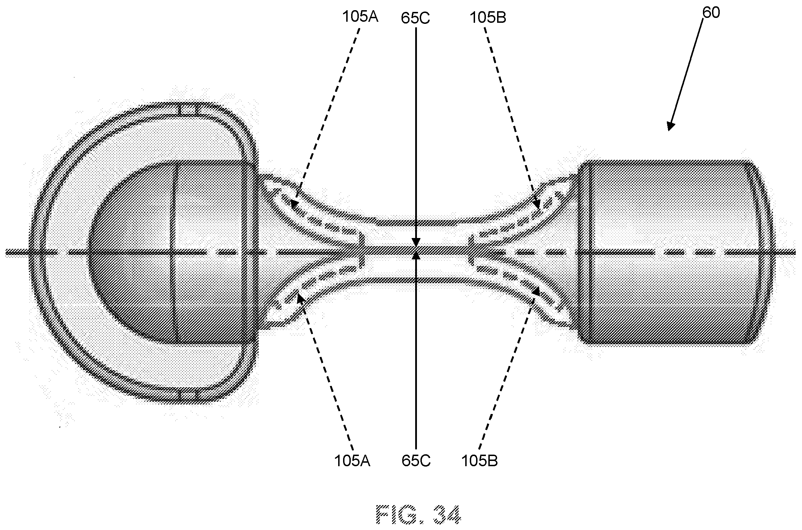

Significantly, by modifying the configuration of notches 105 of pump body 60, NPWT bandage 5 can also function as a pressure limiter, so as to prevent suction source 320 from establishing too high a level of negative pressure in the wound chamber. This is done by forming each of the notches 105 as two interrupted notches 105A, 105B, with a full-thickness side wall portion 65C being disposed between the two interrupted notches 105A, 105B. See Fig. 33. As a result of this construction, when the correct level of negative pressure has been established in the wound chamber, so that pump body 60 transitions from its substantially fully expanded configuration to its substantially fully collapsed configuration, diametrically-opposing full-thickness side wall portions 65C of pump body 60 move radially inwardly and engage one another, thereby sealing inner chamber 70 of pump body 60. See Fig. 34. This prevents suction source 320 from applying any further suction to the wound chamber. In this way, NPWT bandage 5 can also function as a pressure limiter, so as to prevent suction source 320 from establishing too high a level of negative pressure in the wound chamber.

Luer lock 300 may be used with any of the pump assemblies disclosed herein. By way of example but not limitation, luer lock 300 may be used with pump assembly 15 shown in Figs. 1-4, pump assembly 15 shown in Figs. 17-20A, pump assembly 15 shown in Figs. 21-25, pump assembly 15 shown in Figs. 26-28, pump assembly 15 shown in Figs. 29-31, etc.

### NPWT BANDAGE WHEREIN THE WOUND CHAMBER COMPRISES A LATERAL EXTENSION, AND WHEREIN THE PUMP ASSEMBLY IS IN FLUID COMMUNICATION WITH THE LATERAL EXTENSION, SO THAT THE PUMP ASSEMBLY IS LATERALLY OFFSET FROM THE WOUND WHILE REMAINING IN FLUID COMMUNICATION WITH THE WOUND

In some situations, it may be desirable to have the pump assembly laterally offset from the wound while remaining in fluid communication with the wound. More particularly, difficult anatomical regions with more complex geometries present challenges for a negative pressure wound therapy (NPWT) bandage having a pump assembly disposed directly over the wound which is being treated. By providing a NPWT bandage wherein the wound chamber comprises a lateral extension, and wherein the pump assembly is in fluid communication with the lateral extension, the pump assembly may be laterally offset from the wound while remaining in fluid communication with the wound.

By way of example but not limitation, with such a construction, where the NPWT bandage is to be used to treat a wound in the groin area, the NPWT bandage may be positioned on the patient's anatomy so that the primary portion of the wound chamber overlies the wound and the lateral extension of the wound chamber extends along the curve of the thigh so that the pump assembly may be positioned at the top of the upper leg, which is a more stable, and topographically less complicated, anatomical region for supporting the pump assembly.

By way of further example but not limitation, where the NPWT bandage is being used to treat a wound on the patient's back, the NPWT bandage may be positioned on the patient's anatomy so that the primary portion of the wound chamber overlies the wound on the patient's back and the lateral extension of the wound chamber extends along the curve of the patient's torso so that the pump assembly may be positioned on the front of the patient, where it may be easily accessed by the patient.

The NPWT bandage may be provided with a variety of different wound chamber configurations and pump dispositions, as appropriate for different applications.

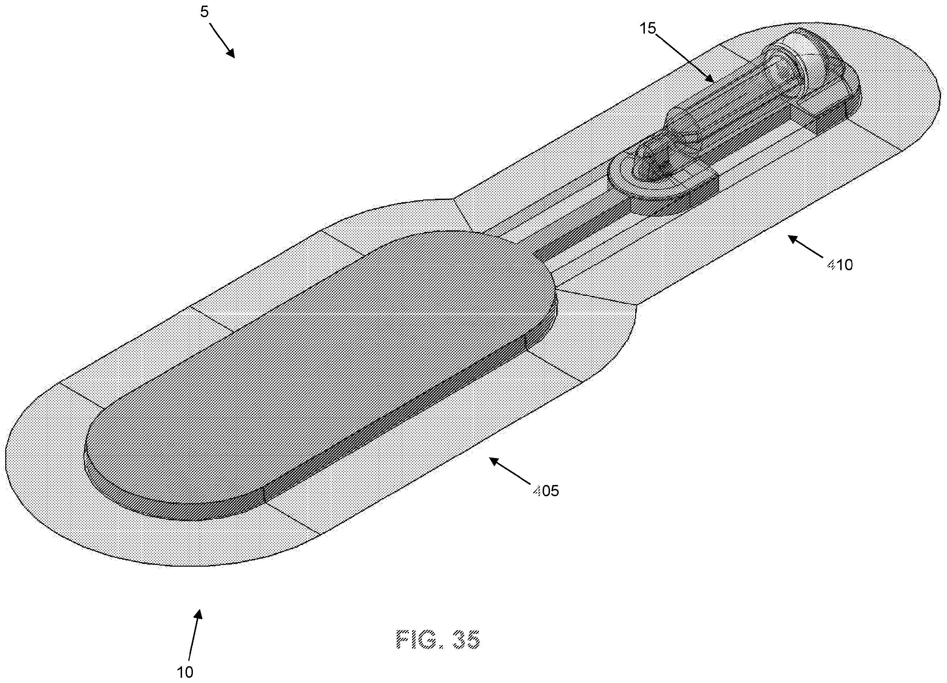

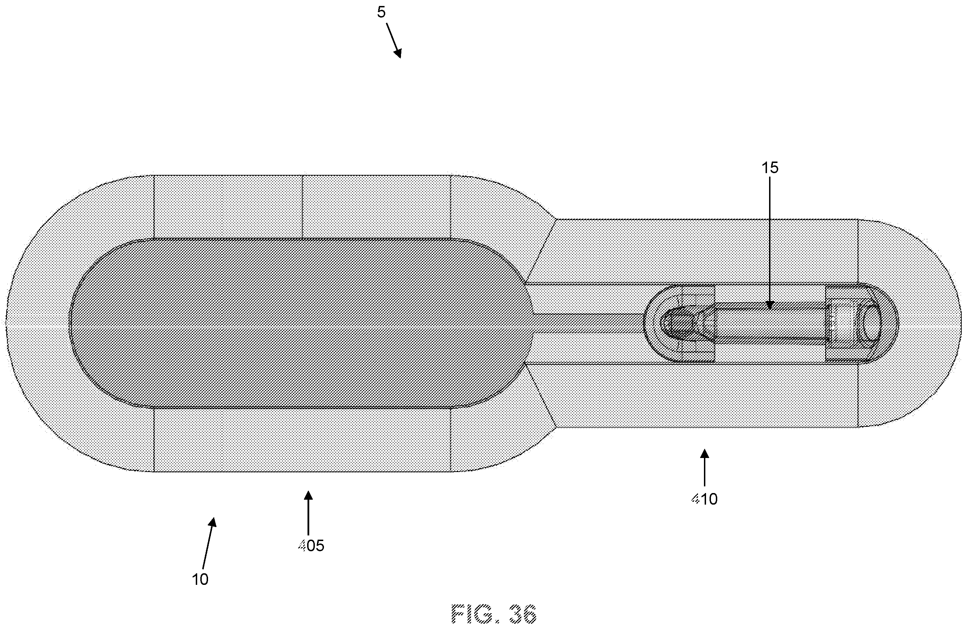

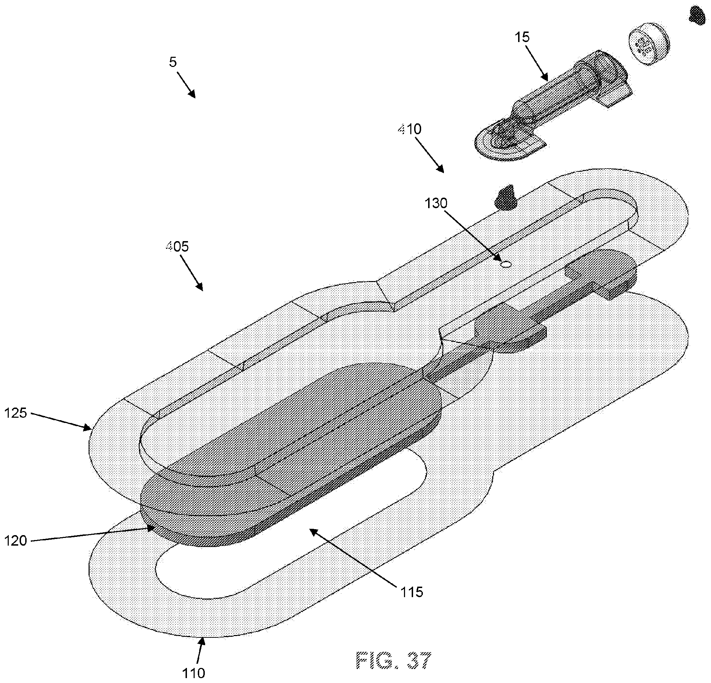

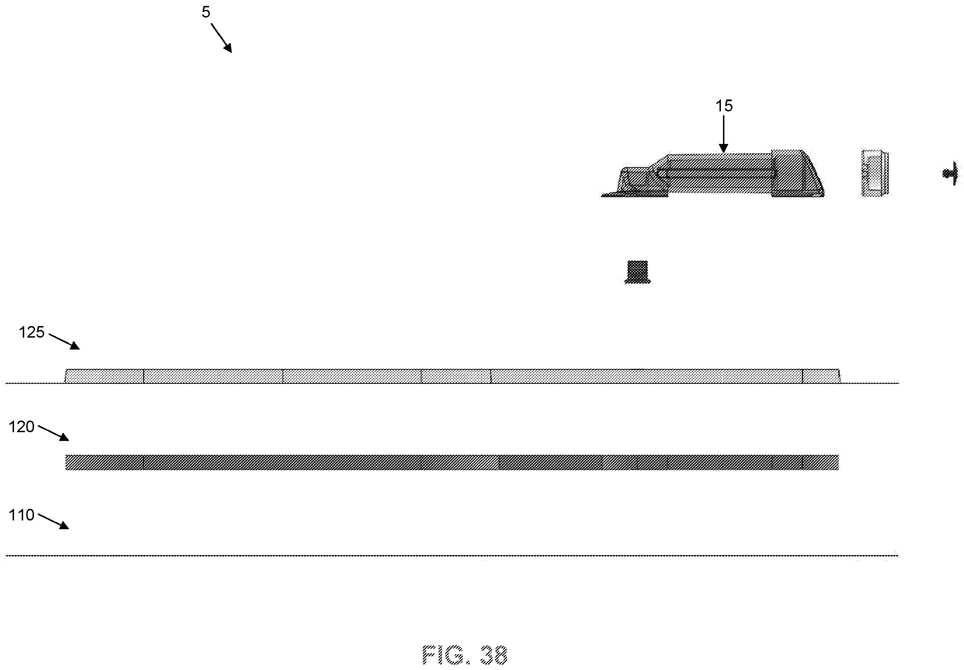

Looking now at Figs. 35-38, there is shown a negative pressure wound therapy (NPWT) bandage 5, wherein the wound chamber comprises a lateral extension, and wherein the pump assembly is in fluid communication with the lateral extension, such that the pump assembly may be laterally offset from the wound while remaining in fluid communication with the wound.

More particularly, the NPWT bandage 5 shown in Figs. 35-38 comprises a membrane 10 and a pump assembly 15. Membrane 10 comprises a first portion 405 for disposition over a wound and a second portion 410 for extending laterally away from the wound and for supporting pump assembly 15 at an anatomical position that is spaced from the wound.

With this form, membrane 10 is generally similar to membrane 10 of the constructions shown in Figs. 21-34 in that membrane 10 comprises a lower skin-contacting polyurethane layer 110, an intermediate foam layer 120 for disposition over lower skin-contacting polyurethane layer 110, and an upper polyurethane layer 125 for disposition over intermediate foam layer 120 and lower skin-contacting polyurethane layer 110. In the preferred form, upper polyurethane layer 125 is formed out of a substantially air-impermeable material. The outer perimeters of upper polyurethane layer 125 and lower skin-contacting polyurethane layer 110 are secured to one another, capturing intermediate foam layer 120 therebetween.

However, in this construction (i.e., the construction shown in Figs. 35-38), center opening 115 is formed in first portion 405 of lower skin-contacting polyurethane layer 110 of membrane 10, and opening 130 is formed in second portion 410 of upper polyurethane layer 125 of membrane 10. Intermediate foam layer 120 is fluid communication with center opening 115 of first portion 405 and with opening 130 of second portion 410. In this way, when NPWT bandage 5 is adhered to anatomy with its center opening 115 positioned over a wound, membrane 10 forms a wound chamber which is generally defined by the configuration of the intermediate foam layer 120. This wound chamber generally comprises (i) a primary portion which overlies center opening 115 of first portion 405, and (ii) a lateral extension which extends from the primary portion of the wound chamber to pump assembly 15.

Pump assembly 15 is mounted to upper polyurethane layer 125 so that wound-side passageway 80 of pump assembly 15 can access fluid (e.g., air, liquid, etc.) within the lateral extension of the wound chamber, and hence access fluid within the primary portion of the wound chamber (i.e., via the porous cells of intermediate foam layer 120), whereby to draw fluid away from the wound site.

In an exemplary use of the NPWT bandage 5 of Figs. 35-38, the NPWT bandage is positioned on the patient's anatomy so that the primary portion of the wound chamber overlies the wound, and the lateral extension of the wound chamber extends along the patient's anatomy so that the pump assembly may be laterally offset from the wound, e.g., positioned at another location which is a more stable, and/or topographically less complicated, and/or more easily accessible for the patient. NPWT bandage 5 may then be used to apply negative pressure to the wound chamber so that fluid (e.g., air, liquid, etc.) can pass from the wound, through center opening 115 into the primary portion of the wound chamber, through the lateral extension of the wound chamber (e.g., through the porous cells of intermediate foam layer 120) and into pump assembly 15 via opening 130 in second portion 410 of upper polyurethane layer 125 of membrane 10.

The NPWT bandage 5 of Figs. 35-38 may also be used at other complicated, or mobile, wound locations, including but not limited to, the foot (diabetic foot ulcers, etc.), a joint such as the ankle, knee, shoulder, or hip (after joint surgeries, ligament repair, arthroscopic procedures, joint replacements, etc.), the arm (lower or upper), the neck, the head, the spine or back (pump assembly 15 can be placed on the front of the body while the primary portion of the wound chamber is positioned over the wound), etc. In each of these wound locations, and others, the anatomical location of the wound can provide a challenge for a wound dressing having an integrated pinch pump assembly, because of the configuration of the anatomy, or the mobility of the anatomical location, or the radius of curvature at the anatomical location, or the location of the wound (which may not be easy for the patient to directly visualize or manually reach), etc. Significantly, an NPWT bandage, wherein the wound chamber comprises a lateral extension, and wherein the pump assembly is in fluid communication with the lateral extension, so that the pump assembly is laterally offset from the wound while remaining in fluid communication with the wound, provides the ability to locate the pinch pump assembly at an easily accessible, and physically stable, anatomical location while still preserving air/fluid communication with the wound.

This NPWT bandage may comprise any number of geometries, including but not limited to:
1 - a wound chamber having a lateral extension of various lengths (e.g., short, long, etc.), wherein the lateral extension is in line with the center of the dressing, with the pinch pump assembly being oriented at any angle to the centerline of the dressing; and
2 - a wound chamber having a lateral extension of various lengths (e.g., short, long, etc.), wherein the lateral extension is located off the centerline of the dressing.

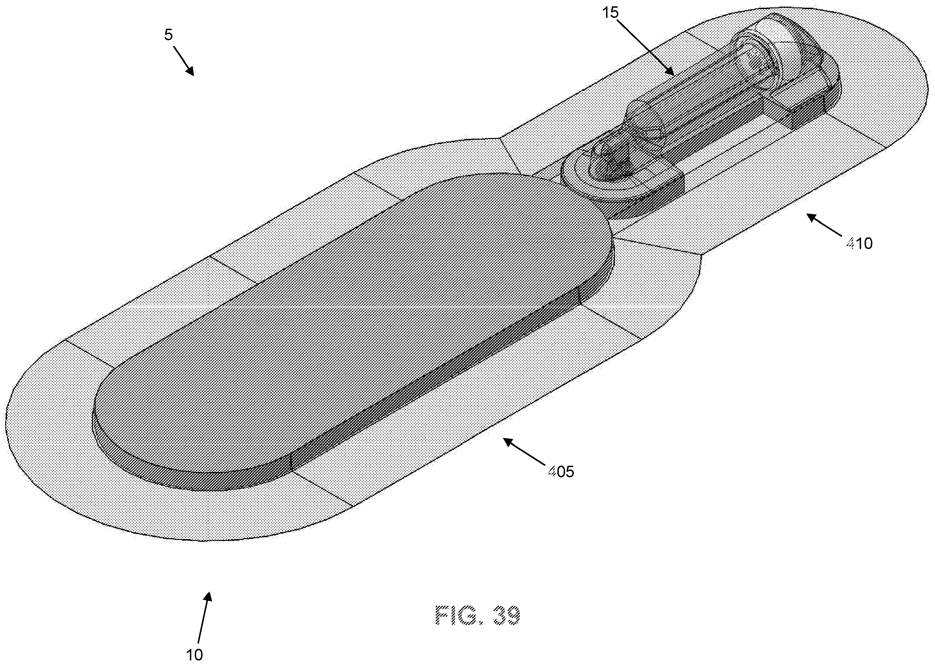

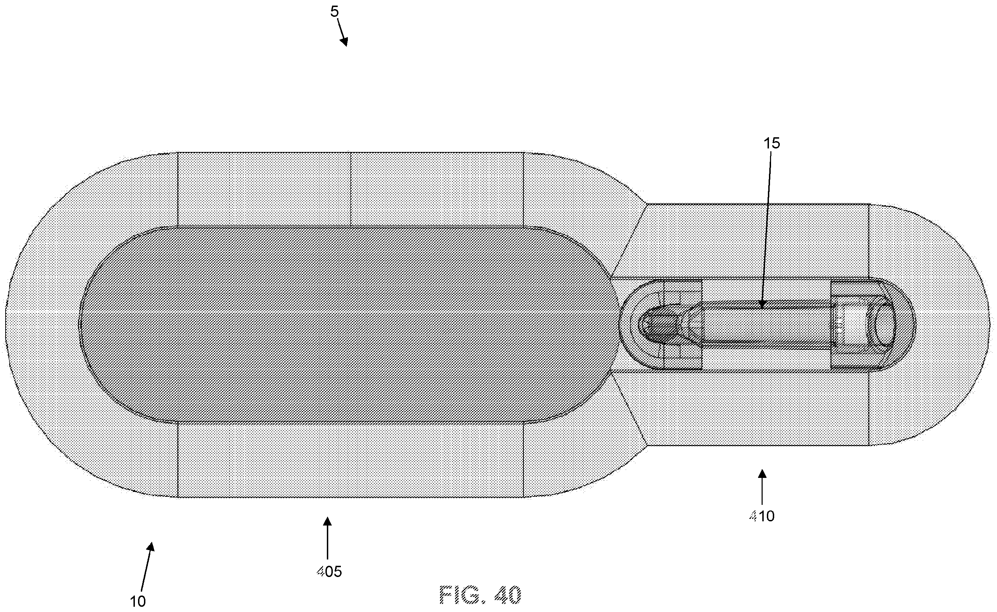

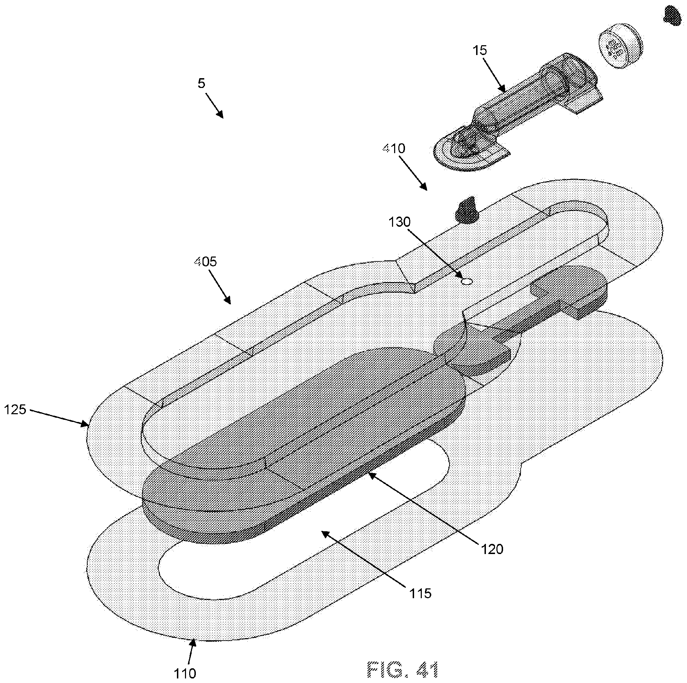

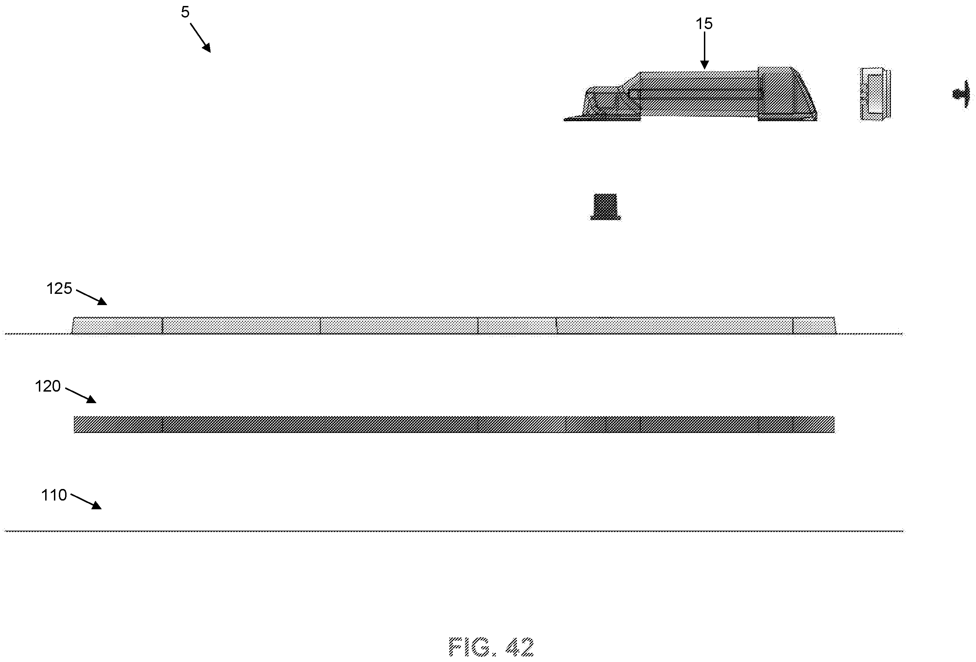

By way of example but not limitation, Figs. 39-42 show an alternative construction for a negative pressure wound therapy (NPWT) bandage 5, wherein the lateral extension of the wound chamber has a different length than that shown in Figs. 35-38.

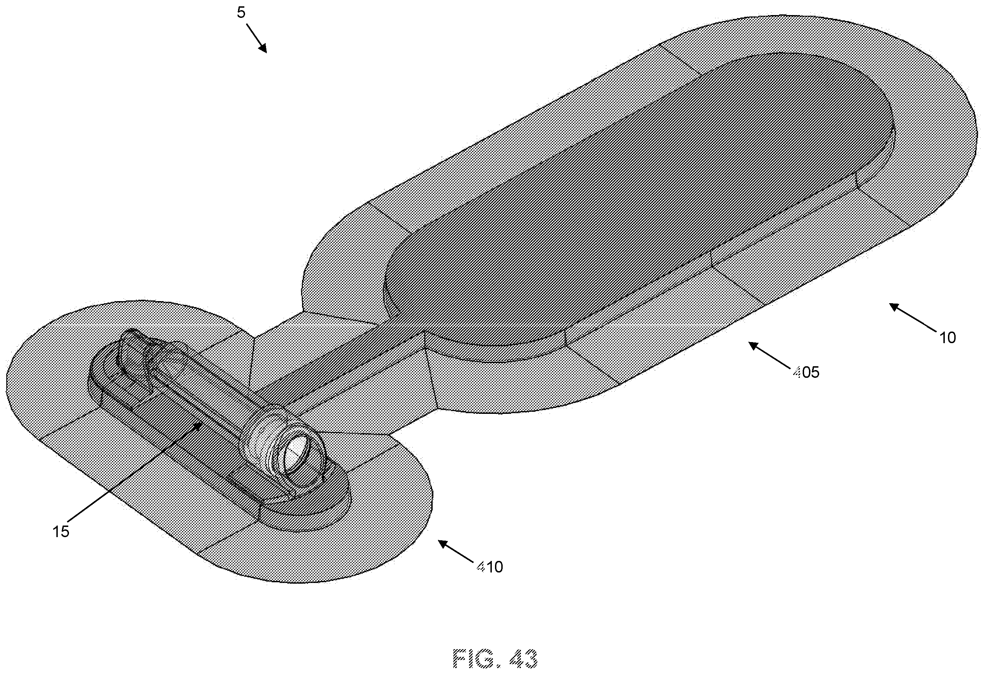

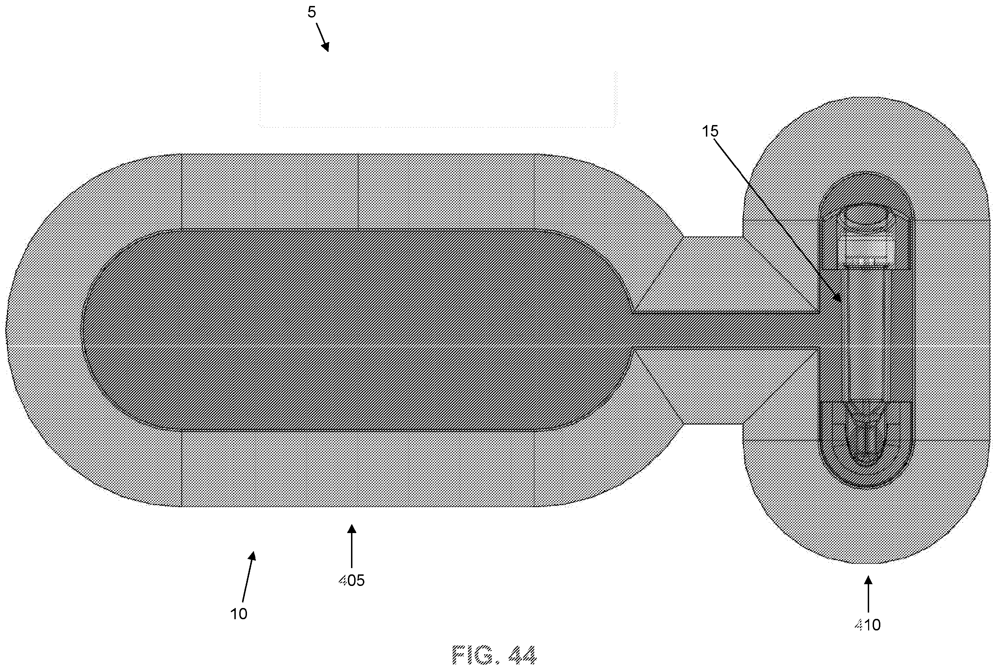

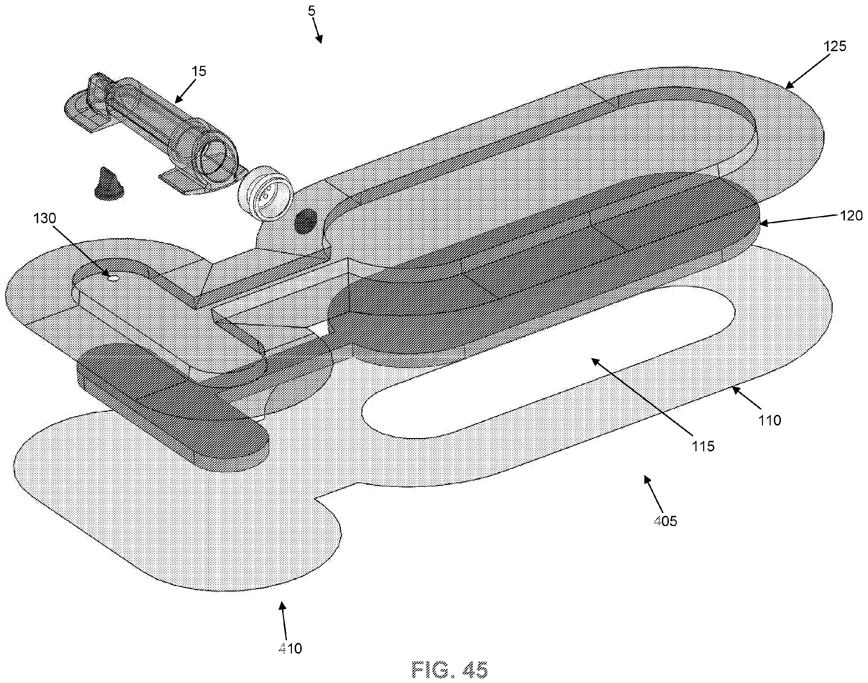

By way of further example but not limitation, Figs. 43-46 show an alternative construction for a negative pressure wound therapy (NPWT) bandage 5, wherein the pinch pump assembly extends transverse to the axis of the lateral extension of the wound chamber.

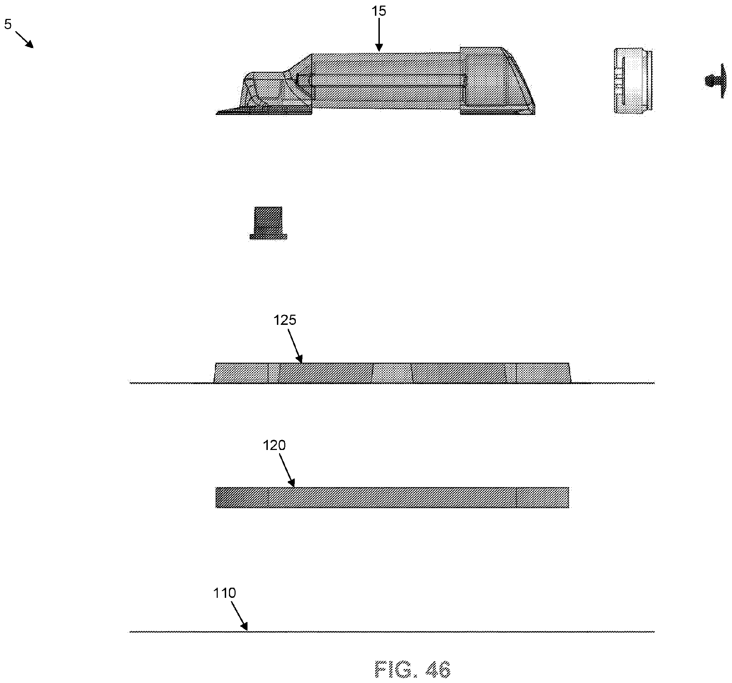

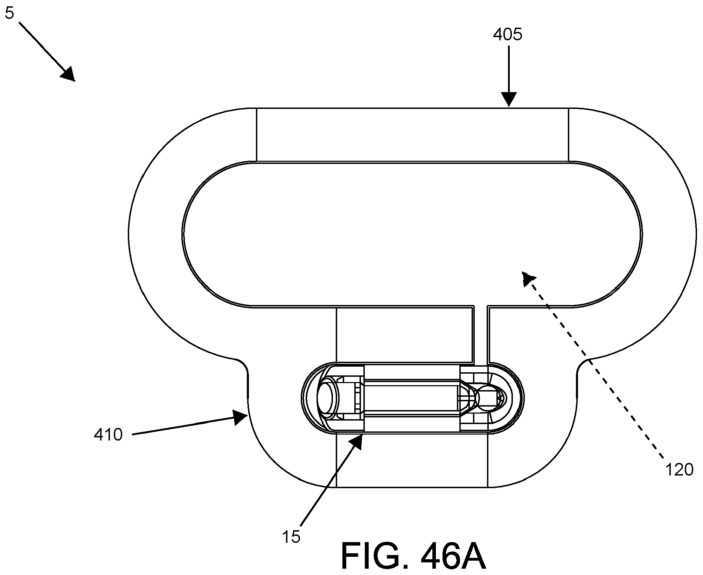

By way of still further example but not limitation, and looking now at Fig. 46A, the second portion 410 of the wound chamber may extend parallel to, but be offset from, first portion 405 of the wound chamber (as opposed to being disposed adjacent to one end of first portion 405 of the wound chamber, such as is shown in the constructions of Figs. 35-38, 39-42 and 43-46). Pump assembly 15 is mounted to second portion 410 so as to be in fluid communication with the wound chamber (in the same manner described for the constructions of Figs. 35-38, 39-42 and 43-46), however, in the construction shown in Fig. 46A, pump assembly 15 extends substantially parallel to first portion 405 of the wound chamber. Note that with the construction of Fig. 46A, the footprint of negative pressure wound therapy (NPWT) bandage 5 may be more of a "square" (in the sense that the length and width of the bandage are more equal) than an "elongated rectangle" (in the sense that the length of the bandage is substantially greater than the width of the bandage) such as is shown in the constructions of Figs. 35-38, 39-42 and 43-46.

If desired, notches 105 formed in pump body 60 of the pump assembly shown in Figs. 17-20 and/or notches 105 formed in pump body 60 of the pump assembly shown in Figs. 26-28 may also be formed into the respective pump bodies of the NPWT bandages 5 shown in Figs. 35-38, Figs. 39-42 and Figs. 43-46. Furthermore, if desired, canister 200 may be used with the pump assemblies of the NPWT bandages 5 shown in Figs. 35-38, Figs. 39-42 and Figs. 43-46. And, if desired, luer lock 300 may be used with the pump assemblies of the NPWT bandages 5 shown in Figs. 35-38, Figs. 39-42 and Figs. 43-46.

### NEGATIVE PRESSURE WOUND THERAPY (NPWT) BANDAGE FOR COVERING A WOUND, WHEREIN THE BANDAGE COMPRISES A TRANSPARENT TOP LAYER WHICH CAPTURES A LAYER OF FOAM AGAINST THE WOUND, WHEREIN THE FOAM LAYER IS PROVIDED WITH A SERIES OF WINDOWS WHICH (i) ALLOW DIRECT VISUALIZATION OF THE WOUND, AND (ii) PROVIDE A METRIC ALONG THE LENGTH OF THE BANDAGE TO INDICATE WHETHER THE BANDAGE NEEDS TO BE REPLACED, AND FURTHER WHEREIN THE FOAM LAYER COMPRISES PERIMETER SERRATIONS WHICH ALLOW THE BANDAGE TO CONFORM TO A CURVED WOUND

The foregoing description discloses a variety of negative pressure wound therapy (NPWT) bandages which cover a wound and apply negative pressure to the wound so as to help prevent infection in the wound. The NPWT bandages disclosed in the foregoing description generally comprise (i) a membrane 10 for disposition over a wound, and (ii) an on-board pump assembly 15 for applying, and maintaining, negative pressure at the wound site.

As shown in the constructions of Figs. 21-46, membrane 10 generally comprises (i) a lower skin-contacting polyurethane layer 110 having an opening 115 for disposition over the wound, (ii) an intermediate foam layer 120 for disposition over opening 115 in lower skin-contacting polyurethane layer 110 so as to be in contact with the wound, and (iii) an upper polyurethane layer 125 for disposition over intermediate foam layer 120 and lower skin-contacting polyurethane layer 110, wherein the outer perimeters of upper polyurethane layer 125 and lower skin-contacting polyurethane layer 110 are secured to one another, capturing intermediate foam layer 120 therebetween, and further wherein lower skin-contacting polyurethane layer 110 is provided with an adhesive for adhering membrane 10 to the skin of a patient.

Pump assembly 15 can be centered over the wound (see pump assembly 15 shown in the embodiments of Figs. 1-25) or can be laterally offset from the wound (see pump assembly 15 shown in the embodiments of Figs. 35-46).

Exudate is frequently released from the wound site, particularly in view of the negative pressure being applied to the wound. In many cases it can be desirable to obtain an indication of the amount of exudate which is being produced by the wound, since high exudate production may require the bandages to be changed more frequently (e.g., because the bandages may become saturated with exudate, or because the exudate may reach the pump, which can diminish pump function and/or cause exudate to be expelled from the pump).

To this end, a new bandage for use with wounds of the type likely to release exudate is now provided. More particularly, and looking now at Figs. 47, 48 and 49, there is shown a bandage 500 for disposition over a wound likely to release exudate. Bandage 500 is similar to the NPWT bandages disclosed in the foregoing description, except that upper polyurethane layer 125 of membrane 10 is replaced with an upper layer 505, and intermediate foam layer 120 of membrane 10 is replaced with foam layer 510.

More particularly, upper layer 505 of bandage 500 is formed out of a transparent material (e.g., a film). The transparent upper layer 505 of the bandage allows an observer to see through the upper layer of the bandage and to observe foam layer 510 (which absorbs the exudate) covering the wound.

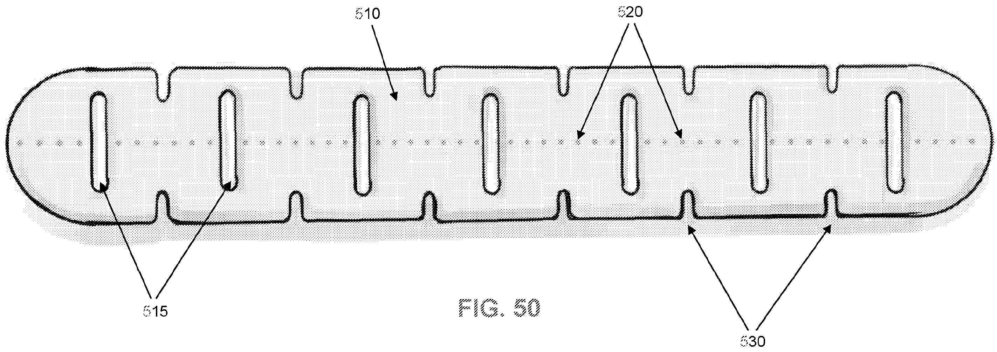

Foam layer 510 (see Figs. 47-55) is provided with a series of windows 515 extending through the foam layer. Windows 515 allow direct visualization of the wound and provide a metric along the length of the bandage to indicate whether the bandage needs to be replaced. Note that in Figs. 50 and 53-55, only foam layer 510 of bandage 500 is shown for clarity of explanation.

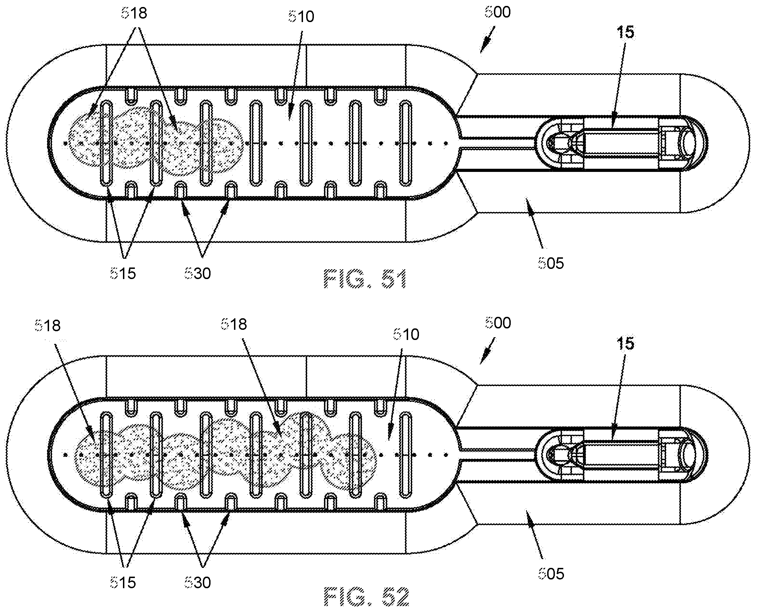

More particularly, and looking now at Figs. 51 and 52, visualization of foam layer 510 through transparent upper layer 505 allows an observer to visualize whether a large amount of exudate has migrated into the foam layer from the wound (the exudate is typically yellowish so it is easily seen against the base color of the foam, which is preferably white, light gray, etc.), and windows 515 provide a metric which extends along the length of the bandage (e.g., each of the windows is a "marker line" along the "ruler" of the bandage). The quantity of exudate emitted by the wound is reflected by the amount of the foam which shows the presence of exudate (this can be quantified by viewing the portions of foam layer 510 which show exudate and counting the number of windows adjacent to the areas of foam layer 510 showing exudate). When the number of windows adjacent to the areas of foam layer 510 showing exudate reaches a certain number along the length of foam layer 510, the observer will have a sense of the quantity of exudate leaving the wound and can decide whether to change the bandage. By way of example but not limitation, if the amount of saturation of foam layer 510 is less than 50% (as shown in Fig. 51, which shows that the number of windows adjacent to the areas of foam layer 510 showing exudate 518 is three out of seven windows), then the bandage may remain on the wound; if, however, the amount of saturation of foam layer 510 is greater than 50% (as shown in Fig. 52, which shows that the number of windows adjacent to the areas of foam layer 510 showing exudate 518 is four or more windows), then the bandage may be changed. By way of further example but not limitation, when the area of foam layer 510 surrounding the window nearest the pump exhibits the presence of exudate, the decision may be made to change the bandage so as to keep the exudate from reaching the pump.

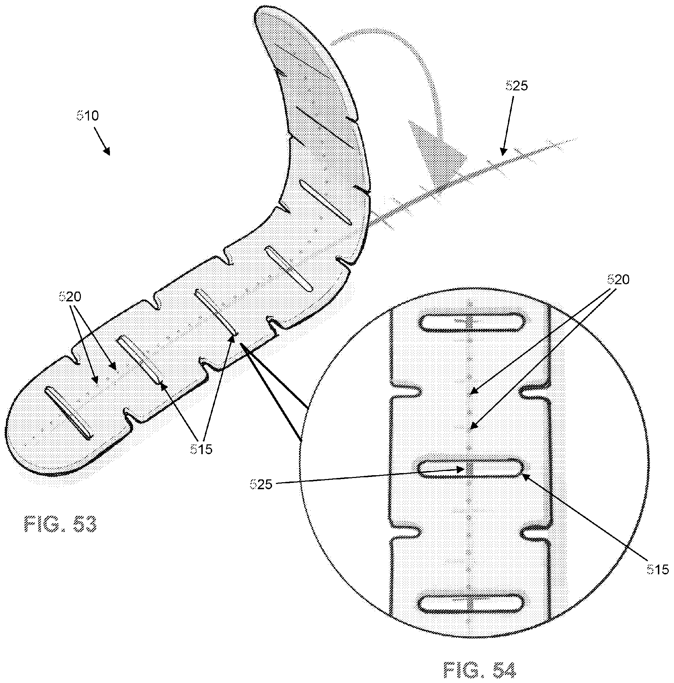

In a preferred embodiment, bandage 500 may be provided with markings 520 along the length of the bandage. Markings 520 may be formed on transparent upper layer 505 and/or foam layer 510 (see Figs. 50-55). As seen in Figs. 53 and 54, markings 520 may be used in combination with windows 515 in foam layer 510 to allow medical personnel to align bandage 500 along a wound line 525 when applying the bandage to the wound.

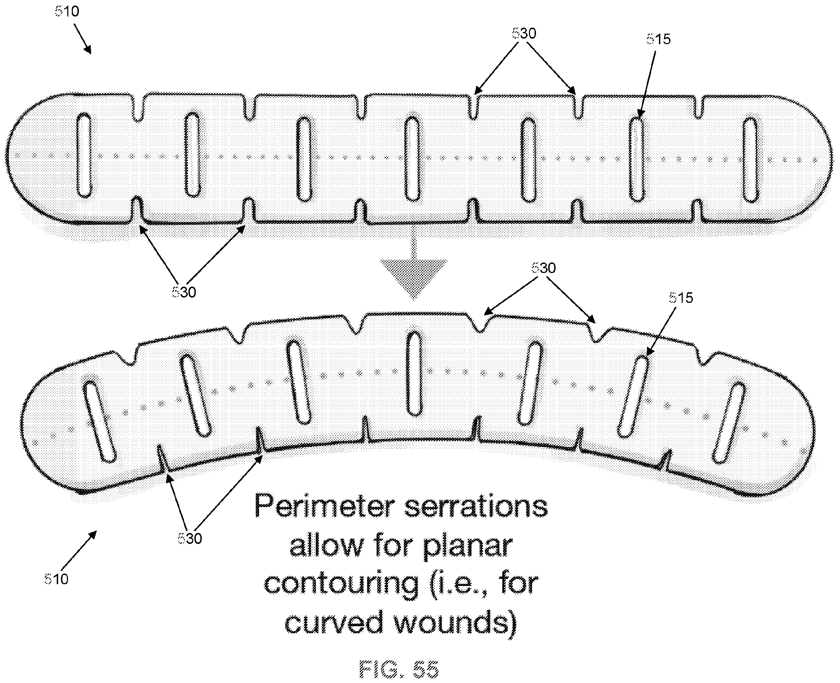

If desired, the perimeter of foam layer 510 may be formed with scalloped edges or perimeter serrations 530 (see Figs. 47-55). As seen in Fig. 55, scalloped edges or perimeter serrations 530 enable bandage 500 to be curved somewhat when being applied to the skin surface so that bandage 500 may be positioned over a non-linear incision. To this end, it is important to note that the portion of bandage 500 that is positioned over a wound must not be curved too much, as too much curvature of the bandage may cause wrinkling of the bandage, which can cause the seal around the wound chamber to fail. The extent of curvature of the bandage that can be accommodated without disrupting the function of the bandage depends on a number of factors, including but not limited to the size and geometry (width and depth) of perimeter serrations 530 in foam layer 510, the ability of the upper layer 505 to deform, the ability of lower skin-contacting polyurethane layer 110 to deform, etc. If desired, perimeter serrations may also be formed in upper layer 505 and/or in lower skin-contacting polyurethane layer 110 so as increase the degree of curvature that the bandage can accommodate (e.g., decrease "wrinkling" of upper layer 505 and lower skin-contacting polyurethane layer 110 when the bandage is positioned over a non-linear incision, so as to increase the degree of curvature that the bandage can accommodate without compromising the air-tight seal made with the skin).

As shown in Figs. 47, 48 and 49, bandage 500 is preferably a negative pressure wound therapy (NPWT) bandage with an on-board pump assembly 15, with pump assembly 15 being laterally offset from bandage 500 in this configuration, which configuration is substantially the same configuration of the NPWT bandage shown in Figs. 35-37. However, if desired, the aforementioned transparent upper layer 505, windows 515, markings 520 and perimeter serrations 530 may be provided with other NPWT bandages having a laterally offset pump assembly, e.g., such as the NPWT bandage shown in Figs. 39-42, and/or the NPWT bandage shown in Figs. 43-46.

As shown in Figs. 56 and 57, bandage 500 is a negative pressure wound therapy (NPWT) bandage with an on-board pump assembly 15, with pump assembly 15 being positioned over a portion of upper layer 505 and foam layer 510.

In still another form, bandage 500 is configured to connect to a pump located remote from the bandage, or to another source of suction (e.g., wall suction).

Lower skin-contacting polyurethane layer 110 of bandage 500 may be omitted, and upper layer 505 may be provided with (i) an adhesive for mounting foam layer 510 to upper layer 505, and (ii) an adhesive for adhering upper layer 505 to the skin of a patient. By way of example but not limitation, the construction of Figs. 47-55 may omit lower skin-contacting polyurethane layer 110 of bandage 500, or the construction of Figs. 56 and 57 may omit lower skin-contacting polyurethane layer 110 of bandage 500, or the construction of Figs. 58, 59 and 60 may omit lower skin-contacting polyurethane layer 110 of bandage 500.

Furthermore, it should be noted that lower skin-contacting polyurethane layer 110 may also be omitted from any of the NPWT bandages shown in Figs. 21-46, with upper polyurethane layer 125 being provided with (i) an adhesive for mounting intermediate foam layer 120 to upper polyurethane layer 125, and (ii) an adhesive for adhering upper polyurethane layer 125 to the skin of a patient.

If desired, notches 105 formed in pump body 60 of the pump assembly shown in Figs. 17-20 and/or notches 105 formed in pump body 60 of the pump assembly shown in Figs. 26-28 may also be formed into the respective pump bodies of the bandage 500 shown in Figs. 47-49 and the bandage 500 shown in Figs. 56 and 57. Furthermore, if desired, canister 200 may be used with the pump assemblies of the bandage 500 shown in Figs. 47-49 and the bandage 500 shown in Figs. 56 and 57. And, if desired, luer lock 300 may be used with the pump assemblies of the bandage 500 shown in Figs. 47-49 and the bandage 500 shown in Figs. 56 and 57.

## Claims

1. A negative pressure wound therapy (NPWT) bandage for applying negative pressure to a wound, the NPWT bandage comprising:
a membrane (10) configured for disposition over a wound so as to form a wound chamber between the membrane and the wound, the membrane comprising a wound-side surface, an atmosphere-side surface, and an opening extending through the membrane from the wound-side surface to the atmosphere-side surface;
a pump assembly (15) carried by the membrane, the pump assembly comprising:
a pump body comprising a wall structure disposed about a pump chamber, wherein at least a portion of the wall structure is resilient;
a wound-side passageway extending through the wall structure and communicating with the wound chamber; and
an atmosphere-side passageway extending through the wall structure and connecting the pump chamber to the atmosphere; and
**characterized in that** the bandage further comprises a canister (200) configured to be in fluid communication with the wound chamber and the pump assembly, the canister comprising an interior chamber for collecting exudates exiting the wound chamber; wherein the pump assembly is configured to generate a negative air pressure within the wound chamber such that exudates from the wound chamber will flow out of the wound chamber and into the interior chamber of the canister.

2. The NPWT bandage of claim 1 wherein the pump assembly and the canister are mounted to the membrane such that exudates flow out of the wound chamber, through the wound-side passageway of the pump assembly, into the pump chamber, through the atmosphere-side passageway of the pump assembly and into the interior chamber of the canister and optionally wherein the pump assembly further comprises an atmosphere-side one-way valve disposed in the atmosphere-side passageway of the pump assembly, the atmosphere-side one-way valve being configured to allow exudates to flow from the pump chamber through the atmosphere-side passageway and into the interior chamber of the canister, but to prevent exudates from flowing through the atmosphere-side passageway from the interior chamber of the canister to the pump chamber.

3. The NPWT bandage of claim 1 wherein the pump assembly and the canister are mounted to the membrane such that exudates flow out of the wound chamber and directly into the interior chamber of the canister and optionally wherein the pump assembly further comprises a wound-side one-way valve disposed in the wound-side passageway of the pump assembly, the wound-side one-way valve being configured to permit fluid to flow through the wound-side passageway from the interior chamber of the canister to the pump chamber, but to prevent fluid from flowing through the wound-side passageway from the pump chamber to the interior chamber of the canister.

4. The NPWT bandage of claim 1 wherein the canister and the pump assembly are mounted to the atmosphere-side surface of the membrane or wherein the canister is mounted to the atmosphere-side surface of the membrane and the pump assembly is mounted to the canister such that the canister is disposed between the atmosphere-side surface of the membrane and the pump assembly.

5. The NPWT bandage of claim 1 wherein the canister further comprises an atmosphere-side opening.

6. The NPWT bandage of claim 5 wherein the atmosphere-side opening comprises a gas-permeable membrane which permits gas to pass through the gas-permeable membrane and optionally wherein the gas-permeable membrane prevents liquid from passing through the gas-permeable membrane.

7. The NPWT bandage of claim 1 wherein the canister is formed integral with the pump body or wherein the canister is formed separately from the pump body.

8. The NPWT bandage of claim 1 wherein the interior chamber of the canister further comprises an absorbent material to absorb liquid present in the interior chamber of the canister and optionally wherein the absorbent material comprises at least one from the group consisting of superabsorbent crystals, hydrocolloids and gel-forming matter.

9. The NPWT bandage of claim 1 wherein the pump assembly comprises at least one of a wound-side one-way valve disposed in the wound-side passageway and an atmosphere-side one-way valve disposed in the atmosphere-side passageway;
wherein the wound-side one-way valve is configured to allow fluid to flow through the wound-side passageway from the wound chamber to the pump chamber but to prevent fluid from flowing through the wound-side passageway from the pump chamber to the wound chamber; and
wherein the atmosphere-side one-way valve is configured to allow fluid to flow through the atmosphere-side passageway from the pump chamber to the atmosphere but to prevent fluid from flowing through the atmosphere-side passageway from the atmosphere to the pump chamber.

## Patentansprüche

1. Unterdruck-Wundtherapie-(NPWT)-Verband zum Aufbringen von Unterdruck auf eine Wunde, wobei der NPWT-Verband Folgendes umfasst:
eine Membran (10), die zur Anordnung über einer Wunde konfiguriert ist, um zwischen der Membran und der Wunde eine Wundkammer zu bilden, wobei die Membran eine wundseitige Oberfläche, eine atmosphärenseitige Oberfläche und eine Öffnung aufweist, die sich von der wundseitigen Oberfläche durch die Membran bis zur atmosphärenseitigen Oberfläche erstreckt;
eine von der Membran getragene Pumpenanordnung (15), wobei die Pumpenanordnung Folgendes umfasst:
ein Pumpenkörper, der eine um eine Pumpenkammer angeordnete Wandstruktur umfasst, wobei mindestens ein Abschnitt der Wandstruktur elastisch ist;
einen wundseitigen Durchgang, der sich durch die Wundwandstruktur erstreckt und mit der Wundkammer kommuniziert; und
einen atmosphärenseitigen Durchgang, der sich durch die Wandkonstruktion erstreckt und die Pumpenkammer mit der Atmosphäre verbindet; und
**dadurch gekennzeichnet, dass** der Verband weiter einen Behälter (200) umfasst, der so konfiguriert ist, dass er mit der Wundkammer und der Pumpenanordnung in strömungstechnischer Kommunikation steht, wobei der Behälter eine Innenkammer zum Auffangen von aus der Wundkammer austretenden Exsudaten umfasst; wobei die Pumpenanordnung so konfiguriert ist, dass sie einen Unterdruck in der Wundkammer erzeugt, sodass Exsudate aus der Wundkammer heraus und in die Innenkammer des Behälters fließen.

2. NPWT-Verband nach Anspruch 1, wobei die Pumpenanordnung und der Behälter so an der Membran montiert sind, dass Exsudate aus der Wundkammer heraus, durch den wundseitigen Durchgang der Pumpenanordnung in die Pumpenkammer, durch den atmosphärenseitigen Durchgang der Pumpenanordnung und in die Innenkammer des Behälters fließen, und optional wobei die Pumpenanordnung weiter ein atmosphärenseitiges Einwegventil im atmosphärenseitigen Durchgang der Pumpenanordnung umfasst, das so konfiguriert ist, dass es zulässt, dass Exsudate aus der Pumpenkammer durch den atmosphärenseitigen Durchgang und in die Innenkammer des Behälters fließen, jedoch verhindert, dass Exsudate aus der Innenkammer des Behälters durch den atmosphärenseitigen Durchgang in die Pumpenkammer fließen.

3. NPWT-Verband nach Anspruch 1, wobei die Pumpenanordnung und der Behälter so an der Membran montiert sind, dass Exsudate aus der Wundkammer heraus und direkt in die Innenkammer des Behälters fließen, und optional wobei die Pumpenanordnung weiter ein wundseitiges Einwegventil umfasst, das im wundseitigen Durchgang der Pumpenanordnung angeordnet ist, wobei das wundseitige Einwegventil so konfiguriert ist, dass es zulässt, dass Flüssigkeit durch den wundseitigen Durchgang von der Innenkammer des Behälters zur Pumpenkammer fließt, jedoch verhindert, dass Flüssigkeit durch den wundseitigen Durchgang von der Pumpenkammer in die Innenkammer des Behälters fließt.

4. NPWT-Verband nach Anspruch 1, wobei der Behälter und die Pumpenanordnung an der atmosphärenseitigen Oberfläche der Membran montiert sind oder wobei der Behälter an der atmosphärenseitigen Oberfläche der Membran montiert ist und die Pumpenanordnung an dem Behälter so montiert ist, dass der Behälter zwischen der atmosphärenseitigen Oberfläche der Membran und der Pumpenanordnung angeordnet ist.

5. NPWT-Verband nach Anspruch 1, wobei der Behälter weiter eine atmosphärenseitige Öffnung umfasst.

6. NPWT-Verband nach Anspruch 5, wobei die atmosphärenseitige Öffnung eine gasdurchlässige Membran umfasst, die Durchtritt von Gas durch die gasdurchlässige Membran zulässt, und optional wobei die gasdurchlässige Membran Durchtritt von Flüssigkeit durch die gasdurchlässige Membran verhindert.

7. NPWT-Verband nach Anspruch 1, wobei der Behälter einstückig mit dem Pumpenkörper gebildet ist, oder wobei der Behälter separat vom Pumpenkörper gebildet ist.

8. NPWT-Verband nach Anspruch 1, wobei die Innenkammer des Behälters weiter ein absorbierendes Material zur Absorption von in der Innenkammer des Behälters vorhandener Flüssigkeit umfasst, und optional wobei das absorbierende Material mindestens eines aus der Gruppe bestehend aus Superabsorberkristallen, Hydrokolloiden und gelbildenden Stoffen umfasst.

9. NPWT-Verband nach Anspruch 1, wobei die Pumpenanordnung mindestens eines von einem wundseitiges Einwegventil, das im wundseitigen Durchgang angeordnet ist, und einem atmosphärenseitigen Einwegventil, das im atmosphärenseitigen Durchgang angeordnet ist, umfasst;
wobei das wundseitige Einwegventil so konfiguriert ist, dass es zulässt, dass Flüssigkeit durch den wundseitigen Kanal von der Wundkammer zur Pumpenkammer fließt, jedoch verhindert, dass Flüssigkeit von der Pumpenkammer durch den wundseitigen Kanal zur Wundkammer fließt; und
wobei das atmosphärenseitige Einwegventil so konfiguriert ist, dass zulässt, dass Flüssigkeit durch den atmosphärenseitigen Durchgang von der Pumpenkammer in die Atmosphäre fließt, aber verhindert, dass Flüssigkeit durch den atmosphärenseitigen Durchgang von der Atmosphäre in die Pumpenkammer fließt.

## Revendications

1. Bandage pour thérapie des plaies par pression négative (NPWT pour Negative Pressure Wound Therapy) destiné à appliquer une pression négative sur une plaie, le bandage NPWT comprenant :
une membrane (10) configurée pour être disposée sur une plaie de manière à former une chambre de plaie entre la membrane et la plaie, la membrane comprenant une surface côté plaie, une surface côté atmosphère et une ouverture s'étendant à travers la membrane de la surface côté plaie à la surface côté atmosphère ;
un ensemble pompe (15) porté par la membrane, l'ensemble de pompe comprenant :
un corps de pompe comprenant une structure de paroi disposée autour d'une chambre de pompe, dans laquelle au moins une partie de la structure de paroi est élastique ;
un passage côté plaie traversant la structure de paroi et communiquant avec la chambre de plaie ; et
un passage côté atmosphère traversant la structure de paroi et reliant la chambre de pompe à l'atmosphère ; et
**caractérisé en ce que** le bandage comprend en outre un récipient (200) configuré pour être en communication fluidique avec la chambre de plaie et l'ensemble pompe, le récipient comprenant une chambre intérieure pour recueillir les exsudats sortant de la chambre de plaie ; dans lequel l'ensemble pompe est configuré pour générer une pression d'air négative à l'intérieur de la chambre de plaie de sorte que les exsudats de la chambre de plaie s'écoulent depuis la chambre de plaie et dans la chambre intérieure du récipient.

2. Bandage NPWT selon la revendication 1, dans lequel l'ensemble pompe et le récipient sont montés sur la membrane de sorte que les exsudats s'écoulent de la chambre de plaie, à travers le passage côté plaie de l'ensemble pompe, dans la chambre de la pompe, à travers le passage côté atmosphère de l'ensemble pompe et dans la chambre intérieure du récipient, et en option dans lequel l'ensemble pompe comprend en outre une valve unidirectionnelle côté atmosphère disposée dans le passage côté atmosphère de l'ensemble pompe, la valve unidirectionnelle côté atmosphère étant configurée pour permettre aux exsudats de s'écouler de la chambre de pompe à travers le passage côté atmosphère et dans la chambre intérieure du récipient, mais pour empêcher les exsudats de s'écouler à travers le passage côté atmosphère de la chambre intérieure du récipient vers la chambre de pompe.

3. Bandage NPWT selon la revendication 1, dans lequel l'ensemble pompe et le récipient sont montés sur la membrane de telle sorte que les exsudats s'écoulent de la chambre de plaie directement dans la chambre intérieure du récipient, et en option dans lequel l'ensemble pompe comprend en outre une valve unidirectionnelle côté plaie disposée dans le passage côté plaie de l'ensemble pompe, la valve unidirectionnelle côté plaie étant configurée pour permettre au fluide de s'écouler à travers le passage côté plaie de la chambre intérieure du récipient vers la chambre de pompe, mais pour empêcher le fluide de s'écouler à travers le passage côté plaie de la chambre de pompe vers la chambre intérieure du récipient.

4. Bandage NPWT selon la revendication 1, dans lequel le récipient et l'ensemble pompe sont montés sur la surface côté atmosphère de la membrane ou dans lequel le récipient est monté sur la surface côté atmosphère de la membrane et l'ensemble pompe est monté sur le récipient de telle sorte que le récipient est disposé entre la surface côté atmosphère de la membrane et l'ensemble pompe.

5. Bandage NPWT selon la revendication 1, dans lequel le récipient comprend en outre une ouverture côté atmosphère.

6. Bandage NPWT selon la revendication 5 dans lequel l'ouverture côté atmosphère comprend une membrane perméable aux gaz qui permet au gaz de passer à travers la membrane perméable aux gaz et, en option dans lequel la membrane perméable aux gaz empêche le liquide de passer à travers la membrane perméable aux gaz.

7. Bandage NPWT selon la revendication 1, dans lequel le récipient est formé d'une seule pièce avec le corps de pompe ou dans lequel le récipient est formé séparément du corps de pompe.

8. Bandage NPWT selon la revendication 1, dans lequel la chambre intérieure du récipient comprend en outre un matériau absorbant destiné à absorber le liquide présent dans la chambre intérieure du récipient et, en option dans lequel le matériau absorbant comprend au moins l'un du groupe consistant en des cristaux superabsorbants, des hydrocolloïdes et une matière gélifiante.

9. Bandage NPWT selon la revendication 1 dans lequel l'ensemble pompe comprend au moins une valve unidirectionnelle côté plaie disposée dans le passage côté plaie et une valve unidirectionnelle côté atmosphère disposée dans le passage côté atmosphère ;
dans lequel la valve unidirectionnelle côté plaie est configurée pour permettre au fluide de s'écouler à travers le passage côté plaie de la chambre de plaie vers la chambre de pompe, mais pour empêcher le fluide de s'écouler à travers le passage côté plaie de la chambre de pompe vers la chambre de plaie ; et
dans lequel la valve unidirectionnelle côté atmosphère est configurée pour permettre au fluide de s'écouler à travers le passage côté atmosphère de la chambre de pompe vers l'atmosphère, mais pour empêcher le fluide de s'écouler à travers le passage côté atmosphère de l'atmosphère vers la chambre de pompe.
